# EUROPEAN PATENT SPECIFICATION

(11) **EP 4 394 027 B1**
(45) Date of publication and mention of the grant of the patent: **30.09.2026**
(21) Application number: 21962825.2
(22) Date of filing: 03.11.2021
(51) Int. Cl.: G01N 21/64, C12M 1/42, C12M 1/00, G02B 6/124, C12Q 1/6869

(54) **DETECTION DEVICE, GENE SEQUENCING SYSTEM, AND DETECTION METHOD**
DETEKTIONSVORRICHTUNG, GENSEQUENZIERUNGSSYSTEM UND DETEKTIONSVERFAHREN
DISPOSITIF DE DÉTECTION, SYSTÈME DE SÉQUENÇAGE GÉNÉTIQUE ET PROCÉDÉ DE DÉTECTION

(43) Date of publication of application: 03.07.2024
(73) Proprietor: MGI Tech Co., Ltd., Shenzhen, Guangdong 518083 (CN)
(72) Inventor: WEI, Yi, Shenzhen, Guangdong 518083 (CN); HUANG, Yi, Shenzhen, Guangdong 518083 (CN); JIANG, Heming, Shenzhen, Guangdong 518083 (CN); YANG, Bin, Shenzhen, Guangdong 518083 (CN); WEN, Xin, Shenzhen, Guangdong 518083 (CN); HUANG, Heng, Shenzhen, Guangdong 518083 (CN); CAO, Mingyou, Shenzhen, Guangdong 518083 (CN); DENG, Qian, Shenzhen, Guangdong 518083 (CN)
(74) Representative: Rentsch Partner AG
(86) International application number: PCT/CN2021/128444
(87) International publication number: WO 2023/077306

(56) References cited:
- CN-A- 107 576 639
- CN-A- 109 596 587
- CN-A- 111 566 528
- CN-A- 112 646 703
- CN-A- 113 155 826
- US-A1- 2017 176 338
- US-B2- 11 029 506

## Description

### FIELD

The present disclosure relates to the technical field of detection, and more particularly, to a detecting apparatus, a gene sequencing system, and a detecting method.

### BACKGROUND

In the field of biochemical detection, it is known to irradiate a sample with a laser to excite the sample to generate fluorescence and detect the generated fluorescence so as to achieve desired detection of the sample. Some related arts will be introduced below by taking gene detection as an example.

The gene detection is completed by exciting a sample by a laser to generate fluorescence, and then detecting and analyzing the generated fluorescence by using an imaging system to obtain a target base sequence. A variety of bases existing on a sample to be detected are labeled by different primers to trace, with the primers being excited by lasers of different wavelengths to obtain corresponding fluorescence (corresponding to the bases in a one-to-one correspondence). In the process of fluorescence excitation, there are accompanying phenomena of photobleaching and fluorescence crosstalk, of which the phenomenon of photobleaching greatly reduces the fluorescence yield and even denaturalizes the fluorescence, resulting in deterioration of the detection result, and the phenomenon of fluorescence crosstalk refers to the following phenomenon: some primers, when being excited to obtain excited fluorescence, are influenced by the excitation process of other primers, causing the quantum yield to be influenced, and multiple fluorescence is generated simultaneously in an area and mixed with each other. The higher the power density of excitation light, the longer the excitation duration and the longer the detection read length, the more obvious the phenomena of photobleaching and fluorescence crosstalk.

In order to pursue high speed, high throughput, and low cost, the laser power density used in the detecting system is getting higher and higher, and the accompanying phenomenon of photobleaching and phenomenon of fluorescence crosstalk seriously affect the sequencing quality and restrict the base sequence length (read length) detectable by the gene detection. In particular, the new generation of high-speed and high-throughput gene detection technology adopts a Time Delay Integration (TDI) imaging technology, which uses the method of lasers of multiple wavelengths simultaneously exciting fluorescence and performing high-speed scanning, and the laser power density used is hundreds of times higher than that adopted in a conventional area array imaging method. With the increase in the detection read length, the influence of the laser of high power density becomes more and more obvious, which leads to the detection quality decreasing faster and faster. The related gene detection technology cannot be better compatible with both "high speed" and "high detection quality and long read length".

In order to reduce the illumination cost and the structural complexity, the existing gene detecting apparatus coaxially outputs lasers of different wavelengths through an optical fiber. When passing through a beam shaping device and an objective lens, main light beams of all wavelengths are coaxially transmitted and finally irradiate on a same area of the sample. At this time, there are two choices, simultaneous excitation and time-division excitation.

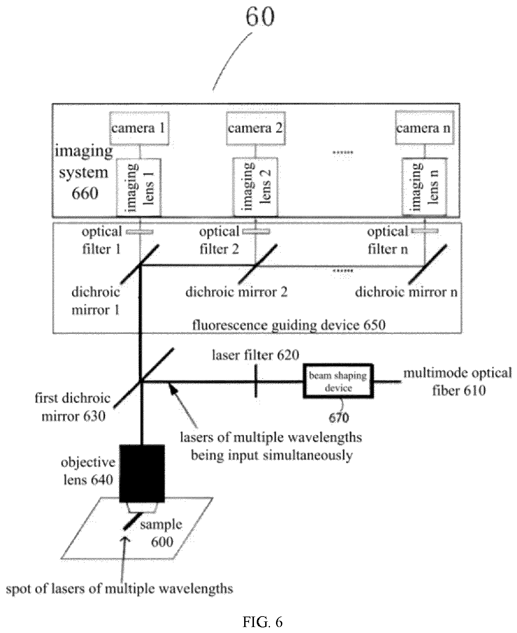

FIG. 6 shows a known apparatus 60 for implementing a first related art (i.e. simultaneous excitation). As shown in FIG. 6, when the first related art (i.e., simultaneous excitation) is adopted, a multimode optical fiber 610 outputs n lasers of different wavelengths simultaneously (n is the number of lasers of different wavelengths, the same below), the lasers are shaped by a beam shaping device 670, filtered by a laser filter 620, reflected by a first dichroic mirror 630, focused by an objective lens 640, etc., and then focused on a sample 600, and the n lasers of different wavelengths are focused on a same position (a position where a spot formed by the lasers of multiple wavelengths in FIG. 6 is) of the sample and excite fluorescence at the position on the sample. Then, the fluorescence is detected by the objective lens 640, then is transmitted through the first dichroic mirror 630, and then passes through further specific dichroic mirrors (i.e., dichroic mirror 1, dichroic mirror 2, ... dichroic mirror n) constituting a fluorescence guiding device 650, and then through specific optical filters (i.e., optical filter 1, optical filter 2, ... optical filter n; each optical filter may only transmit fluorescence of a specific wavelength), so that the fluorescence of different wavelengths is split, and then the fluorescence of different wavelengths passes through imaging lenses (i.e., imaging lens 1, imaging lens 2,... imaging lens n) corresponding to the fluorescence of different wavelengths in an imaging system 660, and then is photographed by cameras (i.e., camera 1, camera 2,... camera n) corresponding to the imaging lenses in the imaging system 660 to output fluorescence information, with the fluorescence excited by the nth laser being transmitted through the nth optical filter corresponding thereto, being focused by the imaging lens n and then being received by the camera n. In this way, the process of simultaneous excitation, and simultaneous detection, of fluorescence can be completed. The detection speed of fluorescence of each Field of View (FOV) is equal to the time for single shot of the camera, and the laser power in the illumination area is the sum of the power of all lasers, that is, the density becomes n times that in the case of irradiation by a single laser (assuming that the laser power density of each wavelength is the same). At a high power density, the fluorescence is more severely influenced by photobleaching, and the phenomenon of fluorescence crosstalk exists among different channels, which both affect the accuracy of detection results. To conclude, the first related art focuses on "high speed", and the method thereof is as follows: the fluorescence is excited by n lasers of different wavelengths simultaneously, the fluorescence detection of all channels can be completed by only one shot in an area of a single FOV, but the power density is n times of that in the case of irradiation by a laser of a single wavelength.

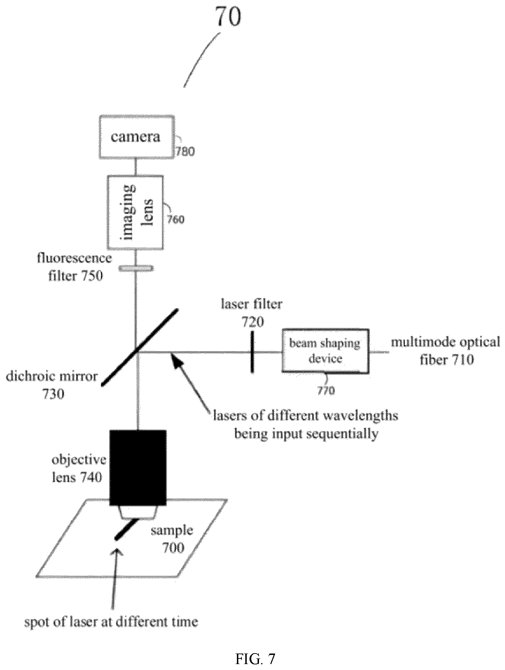

FIG. 7 shows a known apparatus 70 for implementing a second related art (i.e. time-division excitation). As shown in FIG. 7, when the second related art (i.e., time-division excitation) is adopted, a multimode optical fiber 710 sequentially outputs n lasers of different wavelengths, and the output lasers are focused at a position (i.e., the position where a spot of laser at different time in FIG. 7 is) of a sample 700 after being shaped by a beam shaping device 770, filtered by a laser filter 720, reflected by a dichroic mirror 730, and focused by an objective lens 740, etc., and excite fluorescence at the position on the sample. The fluorescence is detected by the objective lens 740, then is transmitted through the dichroic mirror 730, a fluorescence filter 750 (which only transmits the fluorescence and filters out stray light other than the fluorescence), and an imaging lens 760, and then is photographed by a camera 780 to output fluorescence information. Only by n shots, can the fluorescence detection of the FOV be completed. All the lasers need to be output by the multimode optical fiber in sequence and the fluorescence detection is performed in sequence accordingly; that is, the power density of the laser irradiating on the sample is the same as that in the case of irradiation by a single laser (assuming that the power density of each laser is the same). Therefore, the phenomenon of photobleaching is relatively weak and the crosstalk between fluorescence is reduced. However, the fluorescence detection time for each FOV is increased to n times that for a single shot. To conclude, the second related art focuses on "high detection quality and long read length" and the method thereof is as follows: n lasers of different wavelengths excite the fluorescence in turn sequentially (at the same time, cameras corresponding thereto in a one-to-one correspondence take images for fluorescence detection), the power density is the same as that when a single laser excites the fluorescence, but n times of excitation and n times of fluorescence detection are required, the total duration of the fluorescence detection is n times that for the first related art, and the total detection time required for detecting a same sample is n times that for the first related art.

Although the first related art improves the detection speed, the power density of the laser irradiation is increased by n times, different fluorescence is excited at the same position, the phenomenon of photobleaching is obvious, and there is the fluorescence crosstalk, resulting in the decrease of the fluorescence yield. With the increase of the read length, the second related art is obviously superior to the first related art in the detection error rate, but has the shot time become n times as compared with the first related art.

US20170176338A1 discloses that a system may include an illumination system and a detection system, the illumination system may include a light source, a first SLM, at least one lens, and at least one dispersive element, and the detection system may include a 2-D imaging device, at least one lens, and at least one dispersive element. The system may further include a beamsplitter, an objective, and/or a sample holder where a sample to be imaged is placed.

US11029506B2 discloses that an optical beam routing device includes an input port with optical elements and an optical output port formed by an output of an optical element and dichroic reflectors are also part of the optical beam routing device.

Therefore, an improved solution for biochemical detection is needed to solve or alleviate the problems of photobleaching and fluorescence crosstalk caused by the high optical power density, while giving due consideration to the detection speed.

### SUMMARY

The purpose of the present disclosure is to provide an improved solution for biochemical detection, to solve or mitigate the problems of photobleaching and fluorescence crosstalk caused by high optical power density while giving due consideration to the detection speed. The application is defined in the independent claims and the dependent claims.

According to a first aspect of the present disclosure, a detecting apparatus is provided, the detecting apparatus comprising a beam splitting device, a first dichroic mirror, an objective lens, a fluorescence guiding device, and an imaging system including a plurality of imaging devices, wherein,
the beam splitting device is configured to receive and separate an incident light beam of a plurality of different wavelengths from an optical fiber, to form a plurality of excitation light beams corresponding to the plurality of different wavelengths of the incident light beam emitted in different emitting directions from the beam splitting device, wherein, each of the excitation light beams has a wavelength in a one-to-one correspondence with the plurality of different wavelengths of the incident light beam;
the first dichroic mirror is positioned to receive the plurality of excitation light beams corresponding to the plurality of different wavelengths of the incident light beam in a one-to-one correspondence and emitted from the beam splitting device, to transmit the plurality of excitation light beams to the objective lens, such that the plurality of excitation light beams are focused respectively on a plurality of different areas of a sample to be detected through the objective lens, to excite a plurality of fluorescence in the plurality of different areas of the sample on which the plurality of excitation light beams are focused respectively, and to receive the plurality of fluorescence excited by the plurality of excitation light beams respectively and transmit the plurality of fluorescence to the fluorescence guiding device;
the objective lens is positioned to receive the plurality of excitation light beams transmitted by the first dichroic mirror, to focus the plurality of excitation light beams on the plurality of different areas of the sample respectively, and to transmit the plurality of fluorescence excited by the plurality of excitation light beams respectively to the first dichroic mirror;
the fluorescence guiding device is positioned to receive the plurality of fluorescence transmitted by the first dichroic mirror, and to guide the plurality of fluorescence to the plurality of imaging devices respectively, such that each fluorescence of the plurality of fluorescence is imaged by one of the plurality of imaging devices corresponding to the fluorescence; and
each of the plurality of imaging devices is positioned to receive one fluorescence of the plurality of fluorescence guided by the fluorescence guiding device, and to image the fluorescence to obtain fluorescence information corresponding to the fluorescence for detection.

According to a second aspect of the present disclosure, a gene sequencing system is provided, the gene sequencing system comprising an imaging system for collecting a fluorescence signal on a sequencing chip; and an optical system located between the imaging system and the sequencing chip, characterized in that the optical system comprises:
a light source configured to emit an incident light beam of a plurality of different wavelengths;
a beam splitting device configured to receive and separate the incident light beam of a plurality different wavelengths from the light source, to form a plurality of excitation light beams corresponding to the plurality of different wavelengths of the incident light beam emitted in different emitting directions from the beam splitting device, wherein, each of the excitation light beams has a wavelength in a one-to-one correspondence with the plurality of different wavelengths of the incident light beam;
a first dichroic mirror configured to receive the plurality of excitation light beams corresponding to the plurality of different wavelengths of the incident light beam in a one-to-one correspondence and emitted from the beam splitting device;
an objective lens arranged between the sequencing chip and the first dichroic mirror and configured to receive the plurality of excitation light beams transmitted by the first dichroic mirror, to focus the plurality of excitation light beams on a plurality of different areas on the sequencing chip to excite a plurality of fluorescence in the plurality of different areas in one-to-one correspondence with the plurality of excitation light beams respectively, and to transmit the plurality of fluorescence to the first dichroic mirror; and
a fluorescence guiding device configured to receive the plurality of fluorescence transmitted via the first dichroic mirror and to guide the plurality of fluorescence to the imaging system respectively,
wherein the imaging system comprises a plurality of imaging devices corresponding to the plurality of fluorescence in a one-to-one correspondence, each of the plurality of imaging devices being configured to receive one fluorescence, which corresponds to the imaging device, of the plurality of fluorescence guided by the fluorescence guiding device, and to image the fluorescence to obtain fluorescence information corresponding to the fluorescence.

According to a third aspect of the present disclosure, a detecting method is provided, the detecting method comprising:
emitting, by a light source or a light conduction device, an incident light beam of a plurality of different wavelengths;
receiving and separating, by a beam splitting device, the incident light beam of a plurality of different wavelengths from the light source or the light conduction device, to form a plurality of excitation light beams corresponding to the plurality of different wavelengths of the incident light beam emitted in different emitting directions from the beam splitting device, wherein, each of the excitation light beams has a wavelength in a one-to-one correspondence with the plurality of different wavelengths of the incident light beam;
receiving, by a first dichroic mirror, the plurality of excitation light beams corresponding to the plurality of different wavelengths of the incident light beam in a one-to-one correspondence and emitted from the beam splitting device;
receiving, by an objective lens arranged between a sample to be detected and the first dichroic mirror, the plurality of excitation light beams transmitted by the first dichroic mirror, focusing, by the objective lens, the plurality of excitation light beams on a plurality of different areas on the sample to excite a plurality of fluorescence in the plurality of different areas in one-to-one correspondence with the plurality of excitation light beams respectively, and transmitting, by the objective lens, the plurality of fluorescence to the first dichroic mirror; and
receiving, by a fluorescence guiding device, the plurality of fluorescence transmitted by the first dichroic mirror, and guiding, by the fluorescence guiding device, the plurality of fluorescence to a plurality of imaging devices corresponding to the plurality of fluorescence in a one-to-one correspondence, such that each fluorescence of the plurality of fluorescence is received and imaged by one of the plurality of imaging devices corresponding to the fluorescence to obtain fluorescence information corresponding to the fluorescence for detection.

Through an inventive configuration, the present disclosure enables light of different wavelengths to simultaneously and separately illuminate different positions on the sample to be detected, so as to simultaneously excite the fluorescence at the different positions. Taking a laser used as excitation light as an example, the power density of the laser on the sample is reduced to 1/n of the laser density in the case of simultaneous excitation, and the fluorescence detection of all channels can be completed by only a single excitation in an area of a single field of view, and the total detection time is equal to the time required for a single fluorescence excitation and detection when the fluorescence is excited multiple times in a same area for detection (i.e., in the case of time-division excitation). By using the solution of the present disclosure, the power density of the laser on the sample can be reduced without increasing the detection duration, solving the problem that the two aspects of "high speed" and "high detection quality and long read length" cannot be better compatible in the related arts; that is, the problem of photobleaching and fluorescence crosstalk caused by high optical power density can be solved or alleviated, while due consideration can be given to the detection speed.

### BRIEF DESCRIPTION OF THE DRAWINGS

FIG. 1a is a schematic diagram of the composition and structure of a detecting apparatus according to an example of the present disclosure;
FIG. 1b is a schematic diagram of the composition and structure of an exemplary fluorescence guiding device that can be used in a detecting apparatus of the present disclosure;
FIG. 1c is a schematic diagram of the composition and structure of another exemplary fluorescence guiding device that can be used in a detecting apparatus of the present disclosure;
FIG. 2 is a schematic diagram of the composition and structure of a detecting apparatus according to an exemplary embodiment of the present disclosure;
FIG. 3 is a schematic diagram showing a detecting apparatus according to another exemplary embodiment of the present disclosure;
FIG. 4 is a schematic diagram schematically showing spots formed on a surface of a sample by two excitation lasers when detection is performed using a detecting apparatus according to an exemplary embodiment of the present disclosure;
FIG. 5 is a schematic diagram schematically showing a process of performing fluorescence integration scanning in an exemplary embodiment using a TDI imaging technology;
FIG. 6 schematically shows a known apparatus for implementing a related detection technology adopting simultaneous excitation;
FIG. 7 schematically shows a known apparatus for implementing a related detection technology adopting time-division excitation;
FIG. 8a shows a relation between a data quality Q30 and a read length for gene detection results obtained by performing a double-end test on DNA sequences under the same conditions, using a related technology adopting simultaneous excitation, using a related technology adopting time-division excitation, and using the present disclosure; and
FIG. 8b shows a relation between a detection error rate and a read length for gene detection results obtained by performing a double-end test on DNA sequences under the same conditions, using a related technology adopting simultaneous excitation, using a related technology adopting time-division excitation, and using the present disclosure.

### DETAILED DESCRIPTION

The exemplary embodiments of the present disclosure will be described in detail with reference to the accompanying drawings, which form a part of the present disclosure, and which, together with the embodiments of the present disclosure, serve to explain the principle of the present disclosure. For the purpose of clarity and simplification, a detailed specific description of the known functions and structures of the devices described herein will be omitted when it may obscure the subject matter of the present disclosure.

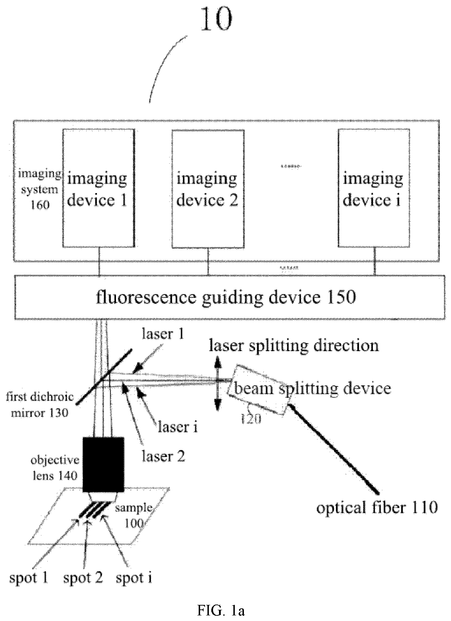

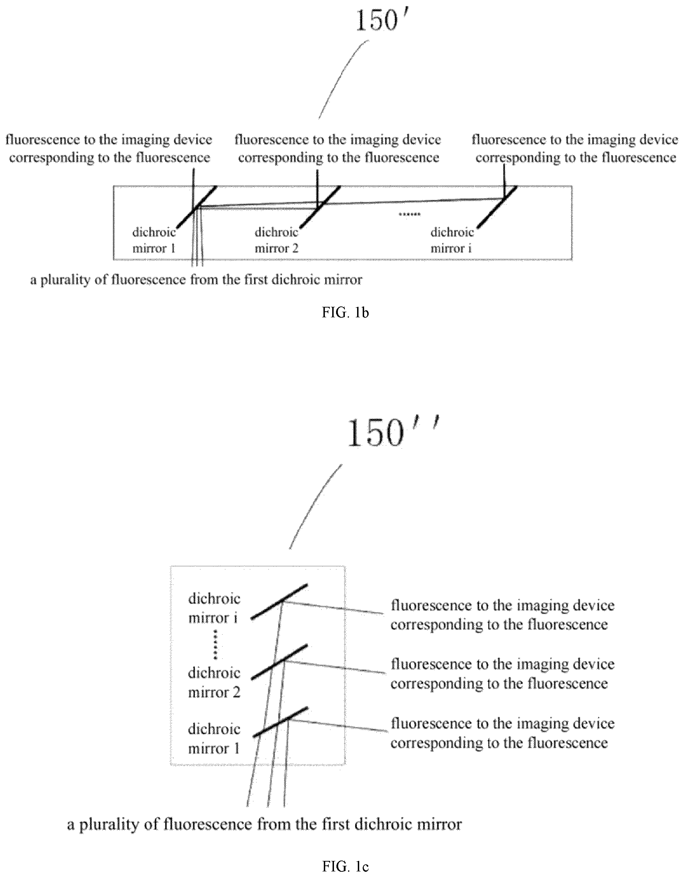

A detecting apparatus 10 according to an example of the present disclosure, as shown in FIG. 1, includes the following components: a beam splitting device 120, a first dichroic mirror 130, an objective lens 140, a fluorescence guiding device 150, and an imaging system 160 including imaging device 1 to imaging device i, where i represents a number of different wavelengths of incident laser(s) from an optical fiber and is an integer greater than 1.

The beam splitting device 120 is configured to receive and separate the incident laser(s) of a plurality of different wavelengths from the optical fiber, such that when the incident laser(s) of the plurality of different wavelengths is/are emitted in different emitting direction(s) from the beam splitting device, each excitation laser corresponding thereto, such as laser 1, laser 2, and laser i shown in FIG. 1, is formed. The wavelength of each excitation laser is the same as the wavelength of the incident laser corresponding to the excitation laser. The beam splitting device may be implemented by various optical devices having a dispersive capability or a combination of such optical devices. For example, the beam splitting device may be, for example, a single dispersion prism, a plurality of dispersion prisms, one or more gratings, and the like, as will be further described later, depending on the specific situation of the actual application and the corresponding requirements. The optical fiber may be a single optical fiber, for example but not limited to a coupled optical fiber. It is advantageous, although not essential, that the incident laser(s) of a plurality of different wavelengths may be incident on the beam splitting device in the same incident direction. Although the incident laser(s) is/are transmitted to the beam splitting device by the optical fiber in FIG. 1, it is possible to provide the incident laser(s) to the beam splitting device with another optical transmission device instead of the optical fiber. The optical fiber or the another optical transmission device may be included in or outside the detecting apparatus.

The first dichroic mirror 130 is positioned to receive the plurality of excitation lasers corresponding to the incident laser(s) of a plurality of different wavelengths respectively and emitted from the beam splitting device, to transmit the excitation laser(s) to the objective lens in such a manner that the plurality of excitation lasers are respectively focused on a plurality of different areas of a sample to be detected 100 (such as positions where spot 1, spot 2, and spot i in FIG. 1 are located) through the objective lens to excite fluorescence in the corresponding areas of the sample on which they are focused respectively, and to receive a plurality of fluorescence that are excited by the plurality of excitation lasers respectively and transmit the plurality of fluorescence to the fluorescence guiding device. In FIG. 1, the focusing spots of laser 1, laser 2, and laser i on the sample are schematically shown by spot 1, spot 2, and spot i. Here, the sample to be detected may be various substances that can emit fluorescence when irradiated by a laser, for example but being not limited to, a biological sample, a chemical sample, and the like. The first dichroic mirror may be suitably oriented in such a manner that the plurality of excitation lasers corresponding to the incident laser(s) of a plurality of different wavelengths respectively and emitted from the beam splitting device are all received by the first dichroic mirror, and after being reflected by the first dichroic mirror, are all focused by the objective lens on the plurality of different areas of the sample, and the plurality of fluorescence that is excited by the plurality of excitation lasers respectively and received from the sample via the objective lens is all transmitted to the fluorescence guiding device via the first dichroic mirror. On this premise, the orientation of the first dichroic mirror may be determined, for example, based on the exit angle of each excitation laser relative to the beam splitting device and the objective lens. Depending on the situation, the first dichroic mirror may be placed at different angles.

The objective lens 140 is positioned to receive the plurality of excitation lasers transmitted via the first dichroic mirror, to focus the plurality of excitation lasers on the plurality of different areas of the sample respectively, and to transmit the plurality of fluorescence excited by the plurality of excitation lasers respectively to the first dichroic mirror. Generally, the objective lens is a lens group composed of a single lens or a combination of multiple lenses. The lens(es) may include, for example, but is not limited to, a convex lens, a concave lens, a glued lens, and the like.

The fluorescence guiding device 150 is positioned to receive the plurality of fluorescence transmitted via the first dichroic mirror and to guide the plurality of fluorescence to the plurality of imaging devices respectively, such that each fluorescence of the plurality of fluorescence is imaged by one of the plurality of imaging devices corresponding to the fluorescence. The fluorescence guiding device may be implemented in various possible ways. For example, the fluorescence guiding device may be a plurality of second dichroic mirrors provided as required and the number of the plurality of second dichroic mirrors may be equal to the number of the excitation lasers, i.e., i. At this time, depending on the situation, each second dichroic mirror may be arranged to achieve one of: transmitting a certain fluorescence and reflecting the remaining fluorescence, and reflecting a certain fluorescence and transmitting the remaining fluorescence, such that the combination of the plurality of second dichroic mirrors achieves the desired guidance of the fluorescence as described above. Each of the plurality of fluorescence guided by the fluorescence guiding device corresponds to each of the plurality of imaging devices in a one-to-one correspondence. According to one possible implementation, the fluorescence guiding device includes a plurality of second dichroic mirrors that are sequentially arranged, the plurality of second dichroic mirrors including a last second dichroic mirror that is away from the first dichroic mirror and at least one preceding second dichroic mirror located between the last second dichroic mirror and the first dichroic mirror, the plurality of fluorescence received by the first dichroic mirror propagating sequentially through the plurality of second dichroic mirrors, each of the preceding second dichroic mirror(s) is positioned to guide one of at least one fluorescence incident thereon among the plurality of fluorescence to a corresponding imaging device, and to guide remaining fluorescence of the at least one fluorescence to a next second dichroic mirror adjacent thereto, and the last second dichroic mirror is positioned to guide fluorescence incident thereon to a corresponding imaging device.

Specifically, for example, referring to an exemplary fluorescence guiding device 150' shown in FIG. 1b, the fluorescence guiding device 150' includes a plurality of second dichroic mirrors that are sequentially arranged: dichroic mirror 1, dichroic mirrors 2, ..., dichroic mirror i. The second dichroic mirror located closest to the first dichroic mirror of the at least one preceding second dichroic mirror among the plurality of second dichroic mirrors, i.e., the dichroic mirror 1, can be selected to transmit the fluorescence that is guided by it to the corresponding imaging device among the plurality of fluorescence from the first dichroic mirror and reflect the remaining fluorescence of the plurality of fluorescence incident thereon to a next second dichroic mirror that is adjacent to it, i.e., the dichroic mirror 2, the last second dichroic mirror of the plurality of second dichroic mirrors, i.e., the dichroic mirror i, is selected to guide the fluorescence incident thereon to the corresponding imaging device through reflection, and for each of the second dichroic mirror(s) in the plurality of second dichroic mirrors other than the second dichroic mirror located closest to the first dichroic mirror and the last second dichroic mirror, for example, the dichroic mirror 2, it is selected to reflect the fluorescence that is guided by it to the corresponding imaging device and transmit the remaining fluorescence of the at least one fluorescence incident thereon to a next second dichroic mirror that is adjacent to it.

As another example, referring to an exemplary fluorescence guiding device 150" shown in FIG. 1c, the fluorescence guiding device 150" includes a plurality of second dichroic mirrors that are sequentially arranged: dichroic mirror 1, dichroic mirror 2, ..., dichroic mirror i. The second dichroic mirror located closest to the first dichroic mirror of the at least one preceding second dichroic mirror among the plurality of second dichroic mirrors, i.e., the dichroic mirror 1, is selected to reflect the fluorescence that is guided by it to the corresponding imaging device among the plurality of fluorescence from the first dichroic mirror and transmit the remaining fluorescence of the plurality of fluorescence incident thereon to a next second dichroic mirror that is adjacent to it, i.e., the dichroic mirror 2, the last second dichroic mirror of the plurality of second dichroic mirrors, i.e., the dichroic mirror i, is selected to guide the fluorescence incident thereon to the corresponding imaging device through reflection, and for each of the second dichroic mirror(s) in the plurality of second dichroic mirrors other than the second dichroic mirror located closest to the first dichroic mirror and the last second dichroic mirror, for example, the dichroic mirror 2, it is selected to reflect the fluorescence that is guided by it to the corresponding imaging device and transmit the remaining fluorescence of the at least one fluorescence incident thereon to a next second dichroic mirror that is adjacent to it.

A dichroic mirror may be positioned in such a manner that light to be received by it is incident thereon at an incident angle in a certain range, which facilitates reflection and/or transmission of the received light by the dichroic mirror, especially in the case of mixed light. The positioning of the dichroic mirror may be determined by considering an upstream component that transmits light to the dichroic mirror. The range may be, for example, an angular range including 45 degrees. Different dichroic mirrors can have different incident angle ranges required by design.

Each of the plurality of imaging devices is positioned to receive a fluorescence corresponding thereto of the plurality of fluorescence guided by the fluorescence guiding device, and to image the fluorescence to obtain fluorescence information corresponding to the fluorescence for detection. The imaging devices may be implemented in various possible ways. According to one possible implementation, each of the plurality of imaging devices includes an optical filter, an imaging lens, and a camera that are sequentially arranged, and for each imaging device, the optical filter thereof is positioned to filter the fluorescence guided to the imaging device by the fluorescence guiding device, and then transmit the filtered fluorescence to the imaging lens thereof, and the imaging lens thereof is positioned to focus the filtered fluorescence transmitted via the optical filter thereof on the camera thereof for imaging by the camera thereof to obtain fluorescence information corresponding to the fluorescence for detection. For each imaging device, including its lens and camera, the center thereof may be kept consistent with the transmission/reflection center of the element corresponding thereto in the fluorescence guiding device, for example, the second dichroic mirror guiding the corresponding fluorescence to the imaging device. The camera may be a TDI camera, which is suitable for TDI imaging.

A plurality of lasers of different wavelengths may be generated by at least one laser source, and the optical fiber may be positioned to receive the plurality of lasers of different wavelengths from the at least one laser source, and further to form and transmit laser(s) of a plurality of different wavelengths to the beam splitting device to achieve the incident laser(s) of a plurality of different wavelengths. The at least one laser source may be included in or outside the detecting apparatus.

Advantageously, the at least one laser source includes a plurality of laser sources, which may be used to generate the plurality of lasers of different wavelengths respectively.

In the example shown in FIG. 1, advantageously, the beam splitting device 120 may be selected in such a manner that an included angle between emitting directions of excitation lasers of adjacent wavelengths among the plurality of excitation lasers emitted from the beam splitting device is not smaller than a threshold angle, such that a difference between incident angles at which the excitation lasers of adjacent wavelengths are incident on the first dichroic mirror is not smaller than a desired angle difference. The desired angular difference may be determined based on a ratio of a minimum spot spacing to a focal length of the objective lens, e.g., is equal to or greater than the ratio of the minimum spot spacing to the focal length of the objective lens. The minimum spot spacing indicates a minimum distance at which focusing spots formed on the sample by the plurality of excitation lasers need to be spaced apart from one another, and may be suitably determined in various possible ways according to the situation. For example, the minimum spot spacing may be related to the requirements of a particular application and may be determined in advance based on the particular application at which the detecting apparatus is directed. For different detecting scenarios, detecting purposes, and/or samples to be detected, the required minimum spacing between the spots may vary. For example, the minimum spot spacing may be determined to satisfy the following conditions: being greater than a size in a corresponding direction of the spot formed on the surface of the sample by the plurality of coupled lasers from the optical fiber without dispersing the plurality of lasers (the size of the narrow side of the spot, i.e., the width of the spot; in the case of FIG. 1, being the size in the lateral direction of the spot shown); and the corresponding direction being a direction along which the spacing between the spaced apart spots is. In the case where the conditions are satisfied, it is advantageous that the value of the minimum spot spacing is as small as possible. Where the value of the minimum spot spacing is relatively large, some of the focused spots and imaging may exceed the range of the field of view of the objective lens, resulting in deterioration of the imaging. For example, in some cases, the minimum spot spacing may be determined to be equal to the height of the field of view of the imaging system on the sample in a particular application. As a simplified way, the threshold angle may be determined based on the ratio of the minimum spot spacing to the focal length of the objective lens. For example, the threshold angle may be determined to be equal to the ratio of the minimum spot spacing to the focal length of the objective lens multiplied by a coefficient, which may be predetermined to take into account considerations, for example, but being not limited to, whether the excitation lasers emitted from the beam splitting device need to undergo shaping/scaling before being received by the first dichroic mirror, the angular magnification that the excitation lasers emitted from the beam splitting device may need to undergo before being received by the first dichroic mirror, and the like. For example, the coefficient may be equal to 1, for example in the absence of a shaping device between the beam splitting device and the first dichroic mirror. As another example, in the case where a shaping device is provided between the beam splitting device and the first dichroic mirror, the coefficient may be determined based on the angular magnification of the shaping device, for example, is equal to the reciprocal of the absolute value of the angular magnification.

Optionally, the detecting apparatus may include at least one of the following shaping devices: a first beam shaping device located between the optical fiber and the beam splitting device, and being positioned to shape the incident laser(s) of a plurality of different wavelengths output from the optical fiber, and to transmit the shaped incident laser(s) to the beam splitting device; and a second beam shaping device located between the beam splitting device and the first dichroic mirror, and being positioned to shape the plurality of excitation lasers corresponding to the plurality of different wavelengths of the incident laser(s) respectively and emitted from the beam splitting device, and to transmit the shaped plurality of excitation lasers to the first dichroic mirror. Each of the first beam shaping device and the second beam shaping device may be implemented in various possible ways. For example, the first beam shaping device may include a first lens group for performing desired shaping and scaling of an incident laser to be incident on the beam splitting device, for example but not limited to causing the incident laser to be incident on the beam splitting device in a manner of forming a spot of a desired shape and/or size; and the second beam shaping device may include a second lens group for performing desired shaping and scaling of an excitation laser emitted from the beam splitting device, for example but not limited to causing the excitation laser to be incident on the first dichroic mirror in a manner of forming a spot of a desired shape and/or size. In addition, for each of the first beam shaping device and the second beam shaping device, the angular magnification thereof can be appropriately determined according to the situation, and the particular composition and structure thereof can be designed based on the desired angular magnification thereof.

According to one possible implementation, the beam splitting device includes a single dispersion prism positioned in such a manner that each of the incident laser(s) of a plurality of different wavelengths from the optical fiber is incident on a first refractive surface of the single dispersion prism and each of the plurality of excitation light beams then exits from a second refractive surface which is different from the first refractive surface, of the single dispersion prism. The incident angles at which the incident laser(s) of a plurality of different wavelengths is/are incident on the first refractive surface of the single dispersion prism may be different and need not to be fixed angles, and may be related to the orientation and placement angle of the single dispersion prism. Advantageously, the single dispersion prism may be positioned in such a manner that the incident laser(s) of a plurality of different wavelengths is/are incident on the first refractive surface of the single dispersion prism at a predetermined incident angle, the predetermined incident angle being selected in such a manner that a deviation angle between an incident direction of the incident laser incident on the first refractive surface and an exit direction of an excitation laser corresponding to the incident light exiting from the second refractive surface is minimized. The single dispersion prism may have a vertex angle formed between the first refractive surface and the second refractive surface, the first refractive surface and the second refractive surface are adjacent surfaces of the single dispersion prism, and the single dispersion prism is selected in such a manner that a combination of the vertex angle of the single dispersion prism and a material selected to form the single dispersion prism enables the included angle to be not smaller than the threshold angle. The single dispersion prism may be, for example, but is not limited to, a triangular prism, for example a right-angle prism having a vertex angle of 45 degrees and made of a material of N-SF11, a quadrangular prism, and the like. For example, in the case of a quadrangular prism being used, a certain angle of the quadrangular prism may be used as a vertex angle, and two adjacent refractive surfaces of the quadrangular prism that define this angle can be used as an incident surface for receiving a laser and an exit surface for outputting the laser respectively to refract the laser, which can be realized by appropriately placing the quadrangular prism. For a given single dispersion prism, the material, the vertex angle, and the like thereof are known, and the predetermined incident angle may be determined by related art means such as table lookup, calculation and the like. In addition, for a given single dispersion prism, for light of any wavelength incident on the prism at such a predetermined incident angle, the dispersive capability of the prism can be calculated by formulas and algorithms available in the related art (for example, "New Concept Physics Course-Optics" by Kaihua ZHAO). For the solution of the present disclosure, as a simplified way, for the plurality of lasers used, light with a certain intermediate wavelength in the wavelength range of the plurality of lasers may be selected, and an amount of a change in the propagation direction of the selected light after the selected light is incident on a certain prism that can be selected at such a predetermined incident angle and refracted by the prism, that is, the minimum deviation angle of the selected light, is calculated; then, the prism with which the minimum deviation angle of the selected light being not smaller than the above threshold angle is satisfied may be determined as a prism that can be used.

According to another possible implementation, the beam splitting device includes a plurality of dispersion prisms. In this case, each of the plurality of dispersion prisms has a vertex angle formed between a first refractive surface and a second refractive surface thereof and is positioned to receive a laser incident thereon with the first refractive surface thereof and cause the laser to exit from the second refractive surface thereof, with a combination of a number of the plurality of dispersion prisms and a vertex angle and a material of each of the plurality of dispersion prisms being selected in such a manner that the included angle is not smaller than the threshold angle. Given the number, placement, and relative positional relation of the dispersion prisms, such a combination may be determined using relevant data, formulas, and algorithms available in the related art, as described above for a single prism. In this case, a first dispersion prism among the plurality of dispersion prisms that first receives the incident laser(s) of a plurality of different wavelengths from the optical fiber may be positioned in such a manner that each of the incident laser(s) of a plurality of different wavelengths from the optical fiber is incident on a first refractive surface of the first dispersion prism and then exits from a second refractive surface of the first dispersion prism, and each of the plurality of dispersion prisms other than the first dispersion prism may be positioned in such a manner that each of the laser(s) exiting from an previous dispersion prism adjacent thereto is incident on a first refractive surface thereof and exits from a second refractive surface thereof. As compared with using a single dispersion prism, when a plurality of dispersion prisms are used, the direction of optical path will be more flexible, and there will be less restriction on the placement position or angle of other components in the detecting apparatus, but the transmittance of laser will be lower when the plurality of dispersion prisms are used. Therefore, in a particular application, one can choose to use a single dispersion prism or a plurality of dispersion prisms according to the actual needs.

As mentioned above, the beam splitting device may also be realized by grating(s). In the case of grating(s) being used, an appropriate combination of the orientation, position, and possible number of the grating(s), etc., may be determined by data (type, parameters, structure, etc. of available gratings), formulas (basic formulas for grating diffraction, etc.), algorithms, etc., available in the related art, such that the beam splitting device satisfies the above-mentioned relevant requirements.

Herein, use of the terms "front"/"rear", "before"/"after", "preceding"/"following", "previous"/"next" is based on the propagation direction of light. For example, in the case where the same light beam propagates through multiple components, a component that the light beam passes through first during propagation of the light beam may be called a front component, a preceding component, or a previous component relative to a component that the light beam passes through later during propagation of the light beam.

The detecting apparatus of the present disclosure is not limited to using laser(s) from an optical fiber as described in the above example, but may use excitation light from various other light sources, such as light emitted by an LED light source, light emitted by a halogen light source, and the like.

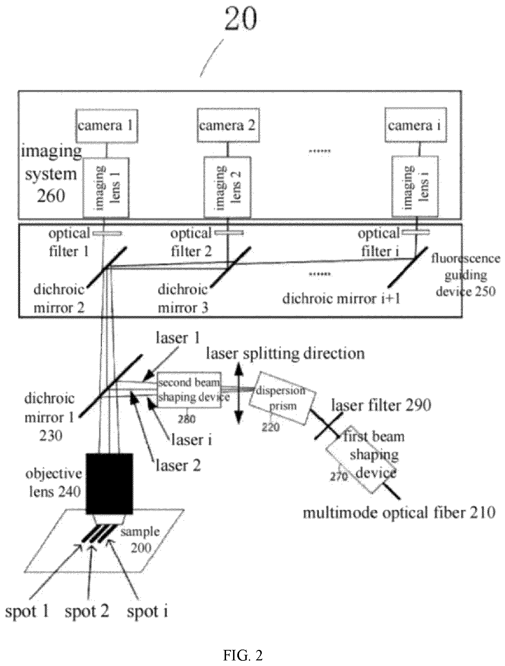

As shown in FIG. 2, a detecting apparatus 20 according to an embodiment of the present disclosure includes the following components: a multimode optical fiber 210, a first beam shaping device 270, a laser filter 290, a dispersion prism 220, a second beam shaping device 280, a dichroic mirror 1 230, an objective lens 240, dichroic mirror 2 to dichroic mirror i+1 and optical filter 1 to optical filter i constituting a fluorescence guiding device 250, and imaging lens 1 to imaging lens i and camera 1 to camera i constituting an imaging system 260, where i represents the type/number of lasers of different wavelengths coupled through the multimode optical fiber, and is an integer greater than 1. It should be understood that for the purpose of clarity and simplification, only lasers of three wavelengths are shown in FIG. 2 to schematically represent lasers of i wavelengths, and the present disclosure is not limited to being only applied to the case of lasers of three wavelengths, but may be applied to the case of lasers of more or fewer wavelengths.

As shown in FIG. 2, the detecting apparatus uses the multimode fiber 210 to coaxially couple i lasers of different wavelengths (i ≥ 2) and then outputs a coupled laser of different wavelengths through the multimode fiber, and the coupled laser is shaped as to the optical path by the first beam shaping device 270, filtered by the laser filter 290, and then incident on a first refractive surface of the dispersion prism 220. According to the law of refraction, the lasers of different wavelengths have different refractive indexes, that is, different refractive angles. Therefore, the lasers of different wavelengths, after being refracted by the first refractive surface of the dispersion prism, will have different propagation angles, and after the lasers of different wavelengths are further refracted by the second refractive surface of the dispersion prism, the difference between the propagation angles of the lasers of different wavelengths will be enlarged. In this way, an included angle greater than zero will be produced between the transmission directions of the lasers of different wavelengths. Generally, the shorter the wavelength of a laser, the greater its corresponding refractive index and refractive angle. After dispersion by the dispersion prism, the i lasers with different propagation angles (schematically shown in FIG. 2 by laser 1, laser 2, and laser i) are reflected by the dichroic mirror 1 230 and focused by the objective lens 240 and then irradiate on the surface of a sample 200. Since the lasers each have different propagation angles, the lasers each form their own spots (schematically shown in FIG. 2 by spot 1, spot 2, and spot i) and irradiate at different positions of the sample, and the laser spot at each of the positions irradiated by the lasers on the sample originates from a laser of a single wavelength.

In order to clearly illustrate the operating process of the detecting apparatus shown in FIG. 2, here assuming that i=3, the apparatus is further exemplified by taking an example of the apparatus using a multimode fiber to couple three lasers of A, B and C (not shown). A coupled laser formed by the three lasers of A, B and C is reflected by the dichroic mirror 1 230 and focused by the objective lens 240 and then irradiate at different positions on the surface of the sample, and at a certain point in time, the three lasers of A, B and C have illumination areas a, b, and c (not shown) on the sample respectively, and excite fluorescence AX, BX and CX (not shown) correspondingly in the areas a, b, and c respectively. Assuming that the area c is foremost, the area a is rearmost, and the area b is in the middle in the moving direction of the sample, the sample scanning direction is the same as the advancing direction of the illumination area (currently, the area a) of the laser A. The three lasers of A, B and C excite fluorescence simultaneously, and at each point in time, only one fluorescence is excited and emitted at each position on the sample that is illuminated. At the next point in time after the sample is moved, the laser A excites fluorescence AX' in a new area, the laser B excites fluorescence BX' in the area a, and the laser C excites fluorescence CX' in the area b. By proceeding back and forth in this way, the scanning of the whole sample can be completed. During the scanning of the sample, all the fluorescence excited at the same time is transmitted to the dichroic mirror 1 via the objective lens, then transmitted to the dichroic mirror 2, guided and filtered via the dichroic mirror 2 to dichroic mirror 4 and the optical filter 1 to optical filter 3, and then imaged by the imaging lens 1 to imaging lens 3 to the camera 1 to camera 3. For example, the dichroic mirror 2 is selected to transmit one of the fluorescence AX, BX, CX transmitted thereto, for example the fluorescence AX, to guide it to the corresponding optical filter 1, and to reflect the remaining fluorescence, for example the fluorescence BX and CX, to the dichroic mirror 3; the dichroic mirror 3 is selected to reflect one of the fluorescence BX and CX transmitted thereto, for example the fluorescence BX, to guide it to the corresponding optical filter 2, and to transmit the remaining fluorescence, for example the fluorescence CX, to the dichroic mirror 4; and the dichroic mirror 4 is selected to reflect the fluorescence transmitted thereto, for example the fluorescence CX, to guide it to the corresponding optical filter 3. Correspondingly, the optical filter 1 may be selected to allow only the fluorescence AX to pass through it to reach the corresponding imaging lens 1; the optical filter 2 may be selected to allow only the fluorescence BX to pass through it to reach the corresponding imaging lens 2; and the optical filter 3 may be selected to allow only the fluorescence CX to pass through it to reach the corresponding imaging lens 3. In this way, different fluorescence excited at the same time corresponds to the camera 1 to camera 3 in a one-to-one correspondence; besides, the fluorescence is excited at different positions on the sample individually, and different fluorescence is separated and captured by different cameras.

Assuming that lasers of n wavelengths are coaxially coupled using a multimode fiber, the duration of single-channel fluorescence detection of each FOV is T, the power of each laser is Wi, and the area of a laser spot on the sample is Si, in the case of the above first related art being adopted, the laser irradiation power density to which a unit area of the sample is subjected is $ρ = {\sum }_{1}^{n} Wi / Si$ ; assuming that *Wi=W, Si=S,* then *ρ = nW*/*S.* According to the operating process as described above, in the case of the technical solution of the present disclosure being adopted, the laser irradiation power density to which a unit area of the sample is subjected is *ρ' = W*/*S = ρ*/*n,* i.e., the laser power density is reduced to 1/n of its original value. Moreover, when the technical solution of the present disclosure is adopted, all the fluorescence can be excited simultaneously and photographed by the cameras at the same time to obtain the fluorescence detection information, and therefore the detection duration of a single FOV is equal to the detection duration T for a single fluorescence in the case of the related art.

For the detecting apparatus shown in FIG. 2, the dispersion prism thereof may be suitably placed at various possible angles and orientations. The placement of the dispersion prism may be determined based at least in part on the dichroic mirror 1, for example, such that the exit angle of an excitation laser that exits from the dispersion prism after being refracted by the dispersion prism conforms to the range of angles of incident light beam that the dichroic mirror 1 is designed to require. With respect to the selection of the dispersion prism (e.g., a material, a vertex angle, etc. of the prism), further exemplary illustration will be given below. Assuming that the focal length of the objective lens in the detecting apparatus is represented by f and the minimum required distance by which the spots formed by different lasers on the sample need to be separated (i.e., the minimum spot spacing) is represented by d, where the approximating condition that "the objective lens is a thin lens and the field of view of the objective lens is <5°" is satisfied, it can be determined that the minimum required angle at which lasers of adjacent wavelengths incident on the dichroic mirror 1 need to be separated is Δθ_{T}≈d/f, and this angle Δθ_{T} may be referred to as a first threshold angle. The dispersive capability of a prism is determined by the material, the size of the vertex angle (the included angle formed by the incident surface and the exit surface of the prism) and the like of the prism. Taking an example of the dispersion prism being a triangular prism, assuming that lasers are incident on the first refractive surface of the triangular prism in the direction that produces the minimum deviation angle, the angle Δθ_{T} can be calculated based on the distance d. With Δθ_{T} determined, it is possible to determine the condition that the included angle between excitation lasers of adjacent wavelengths exiting from the dispersion prism needs to satisfy, that is, the included angle needing to be not smaller than a second threshold angle Δθ_{T}', which can be determined by, for example, considering the angular magnification of the second beam shaping device provided between the beam splitting device and the dichroic mirror 1 and based on the angle Δθ_{T} and the angular magnification, e.g., Δθ_{T}' may be equal to the first threshold angle divided by the absolute value of the angular magnification. A suitable prism may then be selected from available prisms based on the prism dispersion equation in such a manner that the material and the vertex angle of the selected prism enable the included angle between excitation lasers of adjacent wavelengths exiting from the prism to satisfy the relevant requirements. Specifically, it is known from the principles of geometric optics that when a laser is incident on a prism in a direction that produces the minimum deviation angle, the resulting laser image is minimally curved (i.e., the aberration is minimized), and for a laser incident at this incident angle, the angular dispersive ability of the prism, *D_{θ},* is shown by equation (1):
${D}_{θ} = \frac{2 sin \left(α/2\right)}{\sqrt{1 - {n}^{2} {sin}^{2} \left(α/2\right)}} \frac{dn}{dλ} = \frac{b}{a} \frac{dn}{dλ}$
where α represents a vertex angle of the prism, n represents the refractive index of the prism (determined by the material of the prism), λ represents the wavelength of the laser; *dn*/*dλ* represents the dispersive index of the prism, b represents the length of the bottom edge of the prism, and a represents the width of the beam (related to the specific application; different applications require different beam widths).

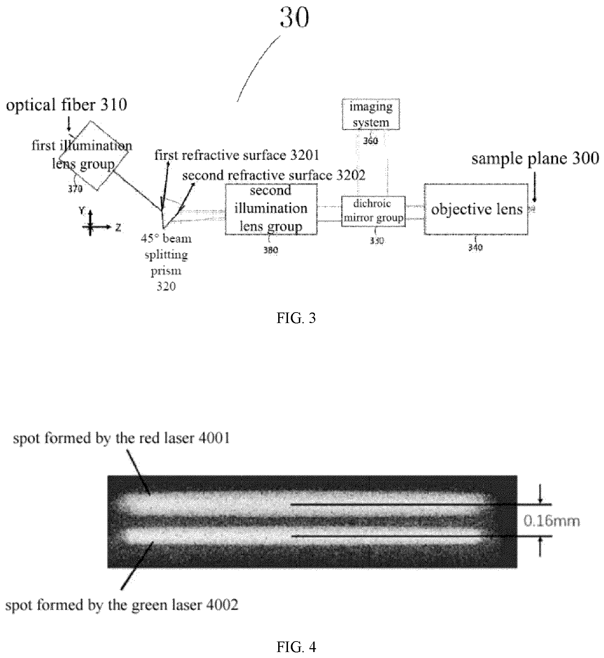

A detecting apparatus 30 according to yet another embodiment of the present disclosure is shown in FIG. 3. Hereinafter, the detecting apparatus of the present disclosure is further exemplarily described by taking an example in which lasers of two wavelengths enter the detecting apparatus shown in FIG. 3 via a coaxial coupled optical fiber, the beam shaping device includes a first illumination lens group 370 and a second illumination lens group 380, and the dispersion prism is a triangular prism 320. The first illumination lens group and the second illumination lens group are combined to form a shaping and scaling system. The dichroic mirror group 330 may correspond, in function and structure, to a combination of the first dichroic mirror and the fluorescence guiding device in FIG. 1 or the dichroic mirror 1 to dichroic mirror i+1 in FIG. 2. The triangular prism is a 45° dispersion prism. An optical path is located in a YZ plane and a sample plane 300 of the sample to be irradiated by the lasers is perpendicular to the YZ plane.

Lasers of two wavelengths, i.e., a green laser and a red laser, are output through a coaxial coupled optical fiber 310. After being shaped by the first illumination lens group 370 and refracted sequentially by a first refractive surface 3201 of the prism 320 and a second refractive surface 3202 of the prism 320, main light beams of the two lasers form an included angle therebetween. The light beams are shaped further by the second illumination lens group 380, and a final included angle between center light beams of the two lasers exiting from the second illumination lens group needs to be not smaller than the above threshold angle Δθ_{T}. The main light beams of the two lasers with different exiting angles are transmitted to an objective lens 340 via dichroic mirrors in the dichroic mirror group 330 and are focused and imaged on the sample by the objective lens, and spots of the two lasers are formed at different positions on the sample and are separated on the sample. On the other hand, fluorescence excited on the sample by the lasers is received by the objective lens 340, and split and guided to an imaging system 360 for imaging by the dichroic mirrors in the dichroic mirror group 330.

The difference between the center wavelengths of the green laser and the red laser is 128nm, and the height of the field of view of the imaging system on the sample is 80µm. Here, a "center wavelength" may be understood as a peak wavelength of a laser. In order to keep the spots of the lasers on the sample separate and non-interfering with each other, the distance between the spots of lasers of adjacent wavelengths on the sample can be preset to be greater than 80µm. The focal length of the objective lens is known, and assuming that the maximum angle of view of the objective lens in the light splitting direction is <5°, i.e., the approximating condition is satisfied, it is determined according to the method described above that Δθ_{T}=0.4°, that is, the included angle |Δθ| between the center light beams of lasers of adjacent wavelengths exiting from the second illumination lens group needs to be greater than 0.4°.

In the YZ plane as shown in FIG. 3, at an approximately central position between the first illumination lens group and the second illumination lens group, the beam diameter of the lasers is approximately 13.5 mm. The distance of the central position from the first illumination lens group is related to the size of the optical fiber and the focal length of the first illumination lens group. Assuming that in this embodiment the central position is about 50mm from the first illumination lens group and after the first illumination lens group, the beam diameter is a beam diameter of the lasers exiting from the first illumination lens group at about 50mm after the first illumination lens group. The second illumination lens group is similar to a telescope in principle, and its angular magnification is M2=-0.7, and by calculation according to the approximating condition (i.e., the objective lens is approximated as a thin lens, and the angle of view of the objective lens is <5°), it can be drawn that the angular difference |Δ*θ*'| between the center light beams of lasers of different wavelengths exiting from the prism and arriving at the second illumination lens group needs to satisfy inequation (2):
$\left|Δθ′\right| \geq \left|Δθ\right| / \left|{M}_{2}\right| \approx 0.57 °$

In the present embodiment, a prism having a vertex angle of 45° and made of a material of N-SF11 is selected. Since an amount of change in dispersive index of most optical materials does not exceed one order of magnitude within the selected laser wavelengths, the dispersive index |*D_{θ}*| of the prism may be calculated for the center wavelength 596nm within the wavelength range of the selected red laser and green laser, and then the so-calculated dispersive index is utilized to judge whether the angular difference between the red laser and green laser used when exiting from the prism satisfies the relevant requirements, thus judging whether the selected prism conforms to the requirements. Specifically, after table lookup and calculation, the following can be obtained:
$λ = 596 nm ⇒ \frac{dn}{dλ} \approx - 1.78 \times {10}^{- 4} \left({nm}^{- 1}\right)$
$\left|{D}_{θ}\right| \approx 0.009 ° / nm$
$\frac{\left|Δθ′\right|}{Δ λ} = 0.57 ° / 128 nm = 0.0045 ° / nm$
where Δλ represents the wavelength difference between the red laser and the green laser. By equations (4) and (5), it can be obtained that |*D_{θ}*| > |Δ*θ*'|/Δλ, i.e., the selected prism satisfies the requirements of the dispersive angle of the present embodiment.

After the material and the vertex angle of the prism are determined, the incident angle of the lasers to the prism may be adjusted to satisfy the condition of the minimum deviation angle. For the embodiment, it is known through calculation that the condition of the minimum deviation angle is satisfied when the incident angle is 43.1°. After optical simulation, it is obtained that the included angle between the main light beams of the lasers of two wavelengths after dispersion by this prism is 1.2°, and when finally reaching the objective lens, the main light beams of the red laser and the green laser form therebetween an included angle of 0.82°, which is > 0.57°, that is, the requirements are satisfied. Finally, the spot distance between the spot formed by the red laser and the spot formed by the green laser on the sample is about 0.16mm. The simulation result is shown in FIG. 4, in which a strip-shaped spot at the upper part is the spot 4001 formed by the red laser, the strip-shaped spot at the lower part is the spot 4002 formed by the green laser, and the centers of the red spot and the green spot are separated by a distance of about 0.16mm.

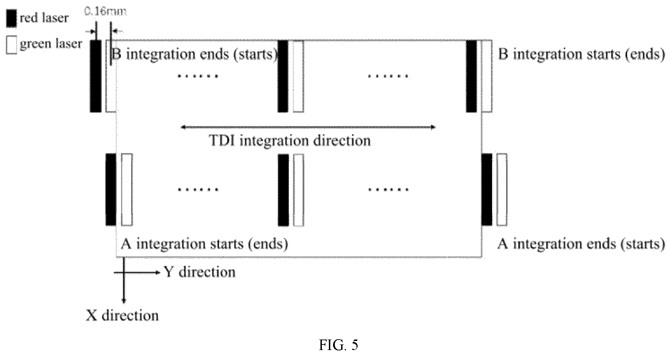

The present embodiment uses the TDI imaging technology, and when each laser is turned on, the corresponding camera is triggered to take images, and the camera integration process when the sample is scanned by the system is shown in FIG. 5. In the process of fluorescence excitation, the red laser and the green laser excite different areas of the sample respectively, and the excitation areas of the laser spots on the sample correspond to the corresponding cameras in a one-to-one correspondence in terms of the object-image relation. Assuming that the red laser corresponds to camera A and the green laser corresponds to camera B. With the TDI imaging technology, the camera integration direction is along the narrow side of the spots, and fluorescence excitation is sequential. When a laser excites the sample, the camera integration is triggered synchronously, that is, the triggering interval between the camera triggering sources corresponding to the red laser and the green laser is equal to the time interval required for a spot to move a distance of 0.16mm relative to the sample (the spot is fixed and the sample is driven by a sliding table located in the XY plane to move). When the green laser enters the sample area, it starts to excite fluorescence, and at the same time, the integration of the camera B starts, and the camera A waits at this time; when the red laser enters the sample, the integration of the camera A starts, and the camera B is integrating at this time; when the green laser leaves the sample, the integration of the camera B ends, and the camera A is still integrating; and the red laser leaves the sample, the integration of the camera A ends, and the fluorescence detection of this row of the sample is completed. Since the time required to move the sample by 0.16 mm is much shorter than the integration duration for a single row, the duration required for all fluorescence detection of the single row is almost equal to the duration of single-channel fluorescence detection. When entering the next row, the red laser and the green laser enter the sample in an opposite order, and integrate in an opposite order. In this way, the scanning detection of the whole sample is completed.

The present disclosure may also be implemented as a detecting system, for example but not limited to a gene sequencing system, including the detecting apparatus as described above. In the case of the gene sequencing system, the imaging system of the detecting apparatus of the present disclosure is used to collect a fluorescent signal on a sequencing chip as a sample to be detected.

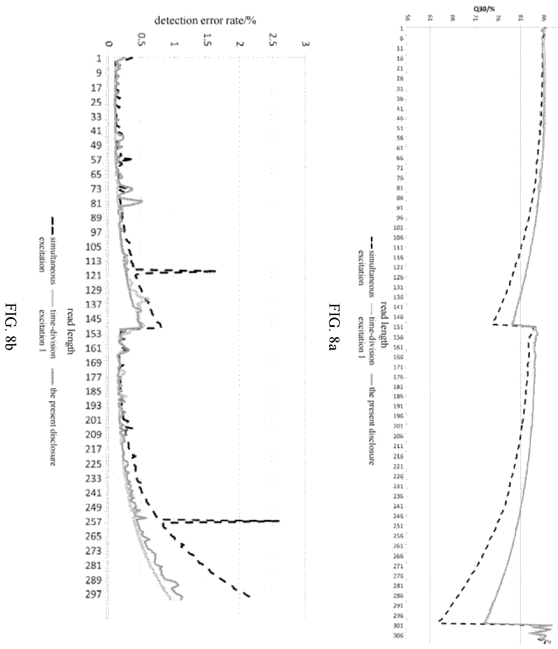

FIG. 8a shows a relation between a data quality Q30 and a read length for gene detection results obtained by performing a double-end test on a test object, i.e., DNA sequences, using the first related art (i.e. simultaneous excitation), using the second related art (i.e., time-division excitation), and using the present disclosure. FIG. 8b shows a relation between a detection error rate and a read length for gene detection results obtained by performing a double-end test on the test object, i.e., DNA sequences, using the first related art (i.e. simultaneous excitation), using the second related art (i.e., time-division excitation), and using the present disclosure. The first related art, the second related art, and the present disclosure are respectively configured for gene detection under the same conditions. In FIG. 8a and FIG. 8b, a dashed line represents a detection result obtained by using the first related art, a hollow line ("time-division excitation 1") represents a detection result obtained by using the second related art, and a solid line ("the present disclosure") represents a detection result obtained by using the present disclosure. In order to compare the technical effects of the first related art, the second related art and the present disclosure, the Q30 and the detection error rate (i.e., the error rate in identifying bases) in the gene detection results are used as evaluation indexes; the higher the Q30, and the lower the detection error rate, the better and the more accurate the detection result is. As can be seen from FIG. 8a and FIG. 8b, the difference between the starting results of the two related arts and the present disclosure at smaller read lengths is very small, but for the first related art, both Q30 and the detection error rate get worse as the read length increases (in general characterized by increasingly smaller Q30 and increasingly higher detection error rate), and for the second related art and the present disclosure, the magnitude of deterioration in Q30 and detection error rate is smaller than that in the case of the first related art; in addition, for both the present disclosure and the second related art, both Q30 and the detection error rate are very close overall, and are the same or substantially the same at some read lengths. Therefore, as far as the gene sequencing results are concerned, the second related art and the present disclosure are similar and both are significantly better than the first related art, but the second related art is doubled as to the time consumed for photographing as compared with the first related art, whereas the detection duration required with the present disclosure is the same as that in the case of the first related art.

The solution of the present disclosure can be applied for various applications requiring fluorescence excitation and fluorescence detection, and is especially suitable for biochemical detection, such as gene detection or other cases requiring exciting of a sample to generate fluorescence and detecting of the excited fluorescence.
The technical means used by the present disclosure in order to achieve the intended purpose and the effect thereof should be more deeply and specifically understood by the detailed description of the specific embodiments; however, the accompanying drawings are merely for reference and illustration; the present invention is only limited by the appended claims.

## Claims

1. A detecting apparatus (10, 20, 30), comprising a beam splitting device (120), a first dichroic mirror (130, 230), an objective lens (140, 240, 340), a fluorescence guiding device (150, 250), and an imaging system (160, 260, 360) comprising a plurality of imaging devices, wherein,
the beam splitting device (120) is configured to receive and separate an incident light beam of a plurality of different wavelengths from an optical fiber (210), to form a plurality of excitation light beams corresponding to the plurality of different wavelengths of the incident light beam emitted in different emitting directions from the beam splitting device (120), wherein, each of the excitation light beams has a wavelength in a one-to-one correspondence with the plurality of different wavelengths of the incident light beam;
the first dichroic mirror (130, 230) is positioned to receive the plurality of excitation light beams corresponding to the plurality of different wavelengths of the incident light beam in a one-to-one correspondence and emitted from the beam splitting device (120), to transmit the plurality of excitation light beams to the objective lens (140, 240, 340), such that the plurality of excitation light beams are focused respectively on a plurality of different areas of a sample (100, 200, 300) to be detected through the objective lens (140, 240, 340), to excite a plurality of fluorescence in the plurality of different areas of the sample (100, 200, 300) on which the plurality of excitation light beams are focused respectively, and to receive the plurality of fluorescence excited by the plurality of excitation light beams respectively and transmit the plurality of fluorescence to the fluorescence guiding device (150, 250);
the objective lens (140, 240, 340) is positioned to receive the plurality of excitation light beams transmitted by the first dichroic mirror (130, 230), to focus the plurality of excitation light beams on the plurality of different areas of the sample (100, 200, 300) respectively, and to transmit the plurality of fluorescence excited by the plurality of excitation light beams respectively to the first dichroic mirror (130, 230);
the fluorescence guiding device (150, 250) is positioned to receive the plurality of fluorescence transmitted by the first dichroic mirror (130, 230), and to guide the plurality of fluorescence to the plurality of imaging devices respectively, such that each fluorescence of the plurality of fluorescence is imaged by one of the plurality of imaging devices corresponding to the fluorescence; and
each of the plurality of imaging devices is positioned to receive one fluorescence of the plurality of fluorescence guided by the fluorescence guiding device (150, 250), and to image the fluorescence to obtain fluorescence information corresponding to the fluorescence for detection,
wherein the beam splitting device (120) is selected in such a manner that an included angle between emitting directions of excitation light beams of adjacent wavelengths among the plurality of excitation light beams emitted from the beam splitting device (120) is not smaller than a threshold angle,
wherein the threshold angle is based on a ratio of a predetermined minimum spacing to a focal length of the objective lens (140, 240, 340), the predetermined minimum spacing indicating a minimum distance at which focusing spots formed on the sample (100, 200, 300) by the plurality of excitation light beams need to be spaced apart from one another,
the detecting apparatus further comprises:
a second beam shaping device (280) located between the beam splitting device (120) and the first dichroic mirror (130, 230), and being positioned to shape the plurality of excitation light beams corresponding to the incident light of a plurality of different wavelengths in a one-to-one correspondence and emitted from the beam splitting device (120), and to transmit shaped plurality of excitation light beams to the first dichroic mirror (130, 230),
wherein the second beam shaping device (280) has an angular magnification, and the threshold angle is equal to a product of the ratio of the predetermined minimum spacing to the focal length of the objective lens (140, 240, 340) multiplied by a coefficient, the coefficient being determined based on the angular magnification of the second beam shaping device (280).

2. The detecting apparatus (10, 20, 30) according to claim 1, further comprising:
at least one light source configured to generate a plurality of excitation light of different wavelengths,
wherein the optical fiber (210) is positioned to receive the plurality of excitation light of different wavelengths from the at least one light source, and to form the incident light beam of a plurality of different wavelengths and transmit the incident light beam of a plurality of different wavelengths to the beam splitting device (120).

3. The detecting apparatus (20) according to claim 1, further comprising:
a first beam shaping device (270) located between the optical fiber (210) and the beam splitting device (120), and being positioned to shape the incident light beam of a plurality of different wavelengths output from the optical fiber (210), and to transmit shaped incident light beam to the beam splitting device (120).

4. The detecting apparatus (10, 20, 30) according to claim 1, wherein the coefficient is equal to a reciprocal of an absolute value of the angular magnification.

5. The detecting apparatus (10, 20, 30) according to claim 1, wherein the beam splitting device (120) comprises at least one dispersion prism (220), or wherein the beam splitting device (120) comprises at least one grating.

6. The detecting apparatus (10, 20, 30) according to claim 5, wherein the at least one dispersion prism (320) comprises a single dispersion prism (320) positioned in such a manner that the incident light beam of a plurality of different wavelengths from the optical fiber (210) is incident on a first refractive surface (3201) of the single dispersion prism (320) and each of the plurality of excitation light beams then exits from a second refractive surface (3202) which is different from the first refractive surface (3201), of the single dispersion prism (320).

7. The detecting apparatus (10, 20, 30) according to claim 6, wherein the incident light beam of a plurality of different wavelengths is incident on the first refractive surface (3201) of the single dispersion prism (320) at a predetermined incident angle, the predetermined incident angle being selected in such a manner that a deviation angle between an incident direction of the incident light beam incident on the first refractive surface (3201) and an exit direction of the plurality of excitation light beams corresponding to the incident light beam and exiting from the second refractive surface (3202) is minimized.

8. The detecting apparatus (10, 20, 30) according to claim 6, wherein the first refractive surface (3201) and the second refractive surface (3202) are adjacent surfaces of the single dispersion prism (320), the single dispersion prism (320) has a vertex angle formed between the first refractive surface (3201) and the second refractive surface (3202), and the single dispersion prism (320) is selected in such a manner that a combination of the vertex angle of the single dispersion prism (320) and a material selected to form the single dispersion prism (320) enables the included angle to be not smaller than the threshold angle.

9. The detecting apparatus (10, 20, 30) according to claim 5, wherein the at least one dispersion prism (320) comprises a plurality of dispersion prisms (320) that are sequentially arranged, each of the plurality of dispersion prisms (320) having a vertex angle formed between a first refractive surface (3201) and a second refractive surface (3202) thereof that are adjacent and being positioned to receive each light incident thereon with the first refractive surface (3201) thereof and cause the light to exit from the second refractive surface (3202) thereof, wherein a combination of a number of the plurality of dispersion prisms (320) and a vertex angle and a material of each of the plurality of dispersion prisms is selected in such a manner that the included angle is not smaller than the threshold angle.

10. The detecting apparatus (10, 20, 30) according to claim 9, wherein a first dispersion prism (320) among the plurality of dispersion prisms (320) that first receives the incident light beam of a plurality of different wavelengths from the optical fiber (210) is positioned in such a manner that the incident light beam of a plurality of different wavelengths from the optical fiber (210) is incident on a first refractive surface (3201) of the first dispersion prism (320) and then exits from a second refractive surface (3202) of the first dispersion prism (320), and each of the plurality of dispersion prisms (320) other than the first dispersion prism (320) is positioned in such a manner that each of light exiting from an previous dispersion prism (320) adjacent thereto is incident on a first refractive surface (3201) thereof and exits from a second refractive surface (3202) thereof.

11. The detecting apparatus (10, 20, 30) according to claim 1, wherein the fluorescence guiding device (150, 250) comprises a plurality of second dichroic mirrors that are sequentially arranged, the plurality of second dichroic mirrors comprising a last second dichroic mirror that is away from the first dichroic mirror (130, 230) and at least one preceding second dichroic mirror located between the last second dichroic mirror and the first dichroic mirror (130, 230), the plurality of fluorescence received by the first dichroic mirror (130, 230) propagating sequentially through the plurality of second dichroic mirrors;
each preceding second dichroic mirror of the preceding second dichroic mirror(s) is positioned to guide one of at least one fluorescence incident thereon among the plurality of fluorescence to the imaging device corresponding to the preceding second dichroic mirror, and to guide remaining fluorescence of the at least one fluorescence to a next second dichroic mirror adjacent to the preceding second dichroic mirror; and
the last second dichroic mirror is positioned to guide fluorescence incident thereon to the imaging device corresponding to the last second dichroic mirror.

12. The detecting apparatus (10, 20, 30) according to claim 11, wherein the second dichroic mirror located closest to the first dichroic mirror (130, 230) among the at least one preceding second dichroic mirror is selected to transmit the fluorescence that is guided thereby to the imaging device corresponding thereto and reflect remaining fluorescence of the plurality of fluorescence incident thereon to a next second dichroic mirror that is adjacent thereto, the last second dichroic mirror is selected to guide the fluorescence incident thereon to the imaging device corresponding to the last second dichroic mirror through reflection; and for each of the second dichroic mirror(s) in the plurality of second dichroic mirrors other than the second dichroic mirror located closest to the first dichroic mirror (130, 230) and the last second dichroic mirror, the second dichroic mirror is selected to reflect the fluorescence that is guided by it to the imaging device corresponding to it and transmit remaining fluorescence of the at least one fluorescence incident thereon to a next second dichroic mirror that is adjacent to it; or
wherein the second dichroic mirror located closest to the first dichroic mirror (130, 230) among the at least one preceding second dichroic mirror is selected to reflect the fluorescence that is guided thereby to the imaging device corresponding thereto and transmit remaining fluorescence of the plurality of fluorescence incident thereon to a next second dichroic mirror that is adjacent thereto, the last second dichroic mirror is selected to guide the fluorescence incident thereon to the imaging device corresponding to the last second dichroic mirror through reflection; and for each of the second dichroic mirror(s) in the plurality of second dichroic mirrors other than the second dichroic mirror located closest to the first dichroic mirror (130, 230) and the last second dichroic mirror, the second dichroic mirror is selected to reflect the fluorescence that is guided by it to the imaging device corresponding to it and transmit remaining fluorescence of the at least one fluorescence incident thereon to a next second dichroic mirror that is adjacent to it.

13. A gene sequencing system, comprising an imaging system (160, 260, 360) for collecting a fluorescence signal on a sequencing chip; and an optical system located between the imaging system (160, 260, 360) and the sequencing chip, **characterized in that** the optical system comprises:
a light source configured to emit an incident light beam of a plurality of different wavelengths;
a beam splitting device (120) configured to receive and separate the incident light beam of a plurality different wavelengths from the light source, to form a plurality of excitation light beams corresponding to the plurality of different wavelengths of the incident light beam emitted in different emitting directions from the beam splitting device (120), wherein, each of the excitation light beams has a wavelength in a one-to-one correspondence with the plurality of different wavelengths of the incident light beam;
a first dichroic mirror (130, 230) configured to receive the plurality of excitation light beams corresponding to the plurality of different wavelengths of the incident light beam in a one-to-one correspondence and emitted from the beam splitting device (120);
an objective lens (140, 240, 340) arranged between the sequencing chip and the first dichroic mirror (130, 230) and configured to receive the plurality of excitation light beams transmitted by the first dichroic mirror (130, 230), to focus the plurality of excitation light beams on a plurality of different areas on the sequencing chip to excite a plurality of fluorescence in the plurality of different areas in one-to-one correspondence with the plurality of excitation light beams respectively, and to transmit the plurality of fluorescence to the first dichroic mirror (130); and
a fluorescence guiding device (150, 250) configured to receive the plurality of fluorescence transmitted via the first dichroic mirror (130, 230) and to guide the plurality of fluorescence to the imaging system (160, 260, 360) respectively,
wherein the imaging system (160, 260, 360) comprises a plurality of imaging devices corresponding to the plurality of fluorescence in a one-to-one correspondence, each of the plurality of imaging devices being configured to receive one fluorescence, which corresponds to the imaging device, of the plurality of fluorescence guided by the fluorescence guiding device (150, 250), and to image the fluorescence to obtain fluorescence information corresponding to the fluorescence,
wherein the beam splitting device (120) is selected in such a manner that an included angle between emitting directions of excitation light beams of adjacent wavelengths among the plurality of excitation light beams emitted from the beam splitting device (120) is not smaller than a threshold angle,
wherein the threshold angle is based on a ratio of a predetermined minimum spacing to a focal length of the objective lens (140, 240, 340), the predetermined minimum spacing indicating a minimum distance at which focusing spots formed on the sequencing chip by the plurality of excitation light beams need to be spaced apart from one another,
the optical system further comprises:
a second beam shaping device (280) located between the beam splitting device (120) and the first dichroic mirror (130, 230), and being positioned to shape the plurality of excitation light beams corresponding to the incident light of a plurality of different wavelengths in a one-to-one correspondence and emitted from the beam splitting device (120), and to transmit shaped plurality of excitation light beams to the first dichroic mirror (130, 230),
wherein the second beam shaping device (280) has an angular magnification, and the threshold angle is equal to a product of the ratio of the predetermined minimum spacing to the focal length of the objective lens (140, 240, 340) multiplied by a coefficient, the coefficient being determined based on the angular magnification of the second beam shaping device (280).

14. A detecting method, comprising:
emitting, by a light source or a light conduction device, an incident light beam of a plurality of different wavelengths;
receiving and separating, by a beam splitting device (120), the incident light beam of a plurality of different wavelengths from the light source or the light conduction device, to form a plurality of excitation light beams corresponding to the plurality of different wavelengths of the incident light beam emitted in different emitting directions from the beam splitting device (120), wherein, each of the excitation light beams has a wavelength in a one-to-one correspondence with the plurality of different wavelengths of the incident light beam;
receiving, by a first dichroic mirror (130, 230), the plurality of excitation light beams corresponding to the plurality of different wavelengths of the incident light beam in a one-to-one correspondence and emitted from the beam splitting device (120);
receiving, by an objective lens (140, 240, 340) arranged between a sample (100, 200, 300) to be detected and the first dichroic mirror (130, 230), the plurality of excitation light beams transmitted by the first dichroic mirror (130, 230), focusing, by the objective lens (140, 240, 340), the plurality of excitation light beams on a plurality of different areas on the sample (100, 200, 300) to excite a plurality of fluorescence in the plurality of different areas in one-to-one correspondence with the plurality of excitation light beams respectively, and transmitting, by the objective lens (140, 240, 340), the plurality of fluorescence to the first dichroic mirror (130, 230); and
receiving, by a fluorescence guiding device (150, 250), the plurality of fluorescence transmitted by the first dichroic mirror (130, 230), and guiding, by the fluorescence guiding device (150, 250), the plurality of fluorescence to a plurality of imaging devices corresponding to the plurality of fluorescence in a one-to-one correspondence, such that each fluorescence of the plurality of fluorescence is received and imaged by one of the plurality of imaging devices corresponding to the fluorescence to obtain fluorescence information corresponding to the fluorescence for detection,
wherein the beam splitting device (120) is selected in such a manner that an included angle between emitting directions of excitation light beams of adjacent wavelengths among the plurality of excitation light beams emitted from the beam splitting device (120) is not smaller than a threshold angle,
wherein the threshold angle is based on a ratio of a predetermined minimum spacing to a focal length of the objective lens (140, 240, 340), the predetermined minimum spacing indicating a minimum distance at which focusing spots formed on the sample (100, 200, 300) by the plurality of excitation light beams need to be spaced apart from one another,
the detecting method further comprises:
shaping, by a second beam shaping device (280) located between the beam splitting device (120) and the first dichroic mirror (130, 230), the plurality of excitation light beams corresponding to the incident light beam of a plurality of different wavelengths in a one-to-one correspondence and emitted from the beam splitting device (120), and transmitting, by the second beam shaping device (280), shaped plurality of excitation light beams to the first dichroic mirror (130, 230),
wherein the second beam shaping device (280) has an angular magnification, and the threshold angle is equal to a product of the ratio of the predetermined minimum spacing to the focal length of the objective lens (140, 240, 340) multiplied by a coefficient, the coefficient being determined based on the angular magnification of the second beam shaping device (280).

## Patentansprüche

1. Eine Detektionsvorrichtung (10, 20, 30), umfassend eine Strahlteilervorrichtung (120), einen ersten dichroitischen Spiegel (130, 230), ein Objektiv (140, 240, 340), eine Fluoreszenzführungsvorrichtung (150, 250) und ein Abbildungssystem (160, 260, 360) mit einer Vielzahl von Abbildungsvorrichtungen, wobei
die Strahlteilervorrichtung (120) dazu konfiguriert ist, einen einfallenden Lichtstrahl einer Vielzahl von unterschiedlichen Wellenlängen von einer optischen Faser (210) zu empfangen und zu trennen, um eine Vielzahl von Anregungslichtstrahlen zu bilden, die der Vielzahl von unterschiedlichen Wellenlängen des einfallenden Lichtstrahls entsprechen, die in unterschiedlichen Emissionsrichtungen von der Strahlteilervorrichtung (120) emittiert werden, wobei jeder der Anregungslichtstrahlen eine Wellenlänge in einer Eins-zu-Eins-Korrespondenz mit der Vielzahl von unterschiedlichen Wellenlängen des einfallenden Lichtstrahls aufweist;
der erste dichroitische Spiegel (130, 230) so positioniert ist, dass er die Vielzahl von Anregungslichtstrahlen empfängt, die der Vielzahl von unterschiedlichen Wellenlängen des einfallenden Lichtstrahls in einer Eins-zu-Eins-Korrespondenz entsprechen und von der Strahlteilervorrichtung (120) emittiert werden, um die Vielzahl von Anregungslichtstrahlen an das Objektiv (140, 240, 340) zu übertragen, so dass die Vielzahl von Anregungslichtstrahlen jeweils auf eine Vielzahl von unterschiedlichen Bereichen einer zu detektierenden Probe (100, 200, 300) durch das Objektiv (140, 240, 340) fokussiert werden, um eine Vielzahl von Fluoreszenzen in der Vielzahl von unterschiedlichen Bereichen der Probe (100, 200, 300) anzuregen, auf die die Vielzahl von Anregungslichtstrahlen jeweils fokussiert werden, und um die Vielzahl von Fluoreszenzen zu empfangen, die jeweils durch die Vielzahl von Anregungslichtstrahlen angeregt werden, und die Vielzahl von Fluoreszenzen an die Fluoreszenzführungsvorrichtung (150, 250) zu übertragen;
das Objektiv (140, 240, 340) so positioniert ist, dass es die Vielzahl von Anregungslichtstrahlen empfängt, die durch den ersten dichroitischen Spiegel (130, 230) übertragen werden, um die Vielzahl von Anregungslichtstrahlen jeweils auf die Vielzahl von unterschiedlichen Bereichen der Probe (100, 200, 300) zu fokussieren und die Vielzahl von Fluoreszenzen, die jeweils durch die Vielzahl von Anregungslichtstrahlen angeregt werden, an den ersten dichroitischen Spiegel (130, 230) zu übertragen;
die Fluoreszenzführungsvorrichtung (150, 250) so positioniert ist, dass sie die Vielzahl von Fluoreszenzen empfängt, die durch den ersten dichroitischen Spiegel (130, 230) übertragen werden, und die Vielzahl von Fluoreszenzen jeweils zu der Vielzahl von Abbildungsvorrichtungen führt, so dass jede Fluoreszenz der Vielzahl von Fluoreszenzen durch eine der Vielzahl von Abbildungsvorrichtungen abgebildet wird, die der Fluoreszenz entspricht; und
jede der Vielzahl von Abbildungsvorrichtungen so positioniert ist, dass sie eine Fluoreszenz der Vielzahl von Fluoreszenzen empfängt, die durch die Fluoreszenzführungsvorrichtung (150, 250) geführt wird, und die Fluoreszenz abbildet, um Fluoreszenzinformationen zu erhalten, die der Fluoreszenz zur Detektion entsprechen,
wobei die Strahlteilervorrichtung (120) so ausgewählt ist, dass ein eingeschlossener Winkel zwischen Emissionsrichtungen von Anregungslichtstrahlen benachbarter Wellenlängen unter der Vielzahl von Anregungslichtstrahlen, die von der Strahlteilervorrichtung (120) emittiert werden, nicht kleiner ist als ein Schwellenwinkel,
wobei der Schwellenwinkel auf einem Verhältnis eines vorbestimmten Mindestabstands zu einer Brennweite des Objektivs (140, 240, 340) basiert, wobei der vorbestimmte Mindestabstand einen Mindestabstand angibt, bei dem Fokusflecken, die auf der Probe (100, 200, 300) durch die Vielzahl von Anregungslichtstrahlen gebildet werden, voneinander beabstandet sein müssen,
die Detektionsvorrichtung ferner umfasst:
eine zweite Strahlformungsvorrichtung (280), die sich zwischen der Strahlteilervorrichtung (120) und dem ersten dichroitischen Spiegel (130, 230) befindet und so positioniert ist, dass sie die Vielzahl von Anregungslichtstrahlen formt, die dem einfallenden Licht einer Vielzahl von unterschiedlichen Wellenlängen in einer Eins-zu-Eins-Korrespondenz entsprechen und von der Strahlteilervorrichtung (120) emittiert werden, und die geformte Vielzahl von Anregungslichtstrahlen an den ersten dichroitischen Spiegel (130, 230) überträgt,
wobei die zweite Strahlformungsvorrichtung (280) eine Winkelvergrösserung aufweist und der Schwellenwinkel gleich einem Produkt des Verhältnisses des vorbestimmten Mindestabstands zur Brennweite des Objektivs (140, 240, 340) multipliziert mit einem Koeffizienten ist, wobei der Koeffizient basierend auf der Winkelvergrösserung der zweiten Strahlformungsvorrichtung (280) bestimmt wird.

2. Die Detektionsvorrichtung (10, 20, 30) nach Anspruch 1, ferner umfassend:
mindestens eine Lichtquelle, die dazu konfiguriert ist, eine Vielzahl von Anregungslicht unterschiedlicher Wellenlängen zu erzeugen,
wobei die optische Faser (210) so positioniert ist, dass sie die Vielzahl von Anregungslicht unterschiedlicher Wellenlängen von der mindestens einen Lichtquelle empfängt und den einfallenden Lichtstrahl einer Vielzahl von unterschiedlichen Wellenlängen bildet und den einfallenden Lichtstrahl einer Vielzahl von unterschiedlichen Wellenlängen an die Strahlteilervorrichtung (120) überträgt.

3. Die Detektionsvorrichtung (20) nach Anspruch 1, ferner umfassend:
eine erste Strahlformungsvorrichtung (270), die sich zwischen der optischen Faser (210) und der Strahlteilervorrichtung (120) befindet und so positioniert ist, dass sie den einfallenden Lichtstrahl einer Vielzahl von unterschiedlichen Wellenlängen, der von der optischen Faser (210) ausgegeben wird, formt und den geformten einfallenden Lichtstrahl an die Strahlteilervorrichtung (120) überträgt.

4. Die Detektionsvorrichtung (10, 20, 30) nach Anspruch 1, wobei der Koeffizient gleich dem Kehrwert eines Absolutwerts der Winkelvergrösserung ist.

5. Die Detektionsvorrichtung (10, 20, 30) nach Anspruch 1, wobei die Strahlteilervorrichtung (120) mindestens ein Dispersionsprisma (220) umfasst, oder wobei die Strahlteilervorrichtung (120) mindestens ein Gitter umfasst.

6. Die Detektionsvorrichtung (10, 20, 30) nach Anspruch 5, wobei das mindestens eine Dispersionsprisma (320) ein einzelnes Dispersionsprisma (320) umfasst, das so positioniert ist, dass der einfallende Lichtstrahl einer Vielzahl von unterschiedlichen Wellenlängen von der optischen Faser (210) auf eine erste Brechungsfläche (3201) des einzelnen Dispersionsprismas (320) einfällt und jeder der Vielzahl von Anregungslichtstrahlen dann aus einer zweiten Brechungsfläche (3202) austritt, die sich von der ersten Brechungsfläche (3201) des einzelnen Dispersionsprismas (320) unterscheidet.

7. Die Detektionsvorrichtung (10, 20, 30) nach Anspruch 6, wobei der einfallende Lichtstrahl einer Vielzahl von unterschiedlichen Wellenlängen auf die erste Brechungsfläche (3201) des einzelnen Dispersionsprismas (320) unter einem vorbestimmten Einfallswinkel einfällt, wobei der vorbestimmte Einfallswinkel so gewählt ist, dass ein Ablenkwinkel zwischen einer Einfallsrichtung des auf die erste Brechungsfläche (3201) einfallenden Lichtstrahls und einer Austrittsrichtung der Vielzahl von Anregungslichtstrahlen, die dem einfallenden Lichtstrahl entsprechen und aus der zweiten Brechungsfläche (3202) austreten, minimiert wird.

8. Die Detektionsvorrichtung (10, 20, 30) nach Anspruch 6, wobei die erste Brechungsfläche (3201) und die zweite Brechungsfläche (3202) benachbarte Flächen des einzelnen Dispersionsprismas (320) sind, das einzelne Dispersionsprisma (320) einen Scheitelwinkel aufweist, der zwischen der ersten Brechungsfläche (3201) und der zweiten Brechungsfläche (3202) gebildet wird, und das einzelne Dispersionsprisma (320) so ausgewählt ist, dass eine Kombination des Scheitelwinkels des einzelnen Dispersionsprismas (320) und eines zur Bildung des einzelnen Dispersionsprismas (320) ausgewählten Materials den eingeschlossenen Winkel nicht kleiner als den Schwellenwinkel sein lässt.

9. Die Detektionsvorrichtung (10, 20, 30) nach Anspruch 5, wobei das mindestens eine Dispersionsprisma (320) eine Vielzahl von Dispersionsprismen (320) umfasst, die sequentiell angeordnet sind, wobei jedes der Vielzahl von Dispersionsprismen (320) einen Scheitelwinkel aufweist, der zwischen einer ersten Brechungsfläche (3201) und einer zweiten Brechungsfläche (3202) davon gebildet wird, die benachbart sind und so positioniert sind, dass sie jedes darauf einfallende Licht mit ihrer ersten Brechungsfläche (3201) empfangen und das Licht aus ihrer zweiten Brechungsfläche (3202) austreten lassen, wobei eine Kombination einer Anzahl der Vielzahl von Dispersionsprismen (320) und eines Scheitelwinkels und eines Materials jedes der Vielzahl von Dispersionsprismen so ausgewählt ist, dass der eingeschlossene Winkel nicht kleiner als der Schwellenwinkel ist.

10. Die Detektionsvorrichtung (10, 20, 30) nach Anspruch 9, wobei ein erstes Dispersionsprisma (320) unter der Vielzahl von Dispersionsprismen (320), das zuerst den einfallenden Lichtstrahl einer Vielzahl von unterschiedlichen Wellenlängen von der optischen Faser (210) empfängt, so positioniert ist, dass der einfallende Lichtstrahl einer Vielzahl von unterschiedlichen Wellenlängen von der optischen Faser (210) auf eine erste Brechungsfläche (3201) des ersten Dispersionsprismas (320) einfällt und dann aus einer zweiten Brechungsfläche (3202) des ersten Dispersionsprismas (320) austritt, und jedes der Vielzahl von Dispersionsprismen (320) ausser dem ersten Dispersionsprisma (320) so positioniert ist, dass jedes Licht, das aus einem vorhergehenden, daran angrenzenden Dispersionsprisma (320) austritt, auf eine erste Brechungsfläche (3201) davon einfällt und aus einer zweiten Brechungsfläche (3202) davon austritt.

11. Die Detektionsvorrichtung (10, 20, 30) nach Anspruch 1, wobei die Fluoreszenzführungsvorrichtung (150, 250) eine Vielzahl von zweiten dichroitischen Spiegeln umfasst, die sequentiell angeordnet sind, wobei die Vielzahl von zweiten dichroitischen Spiegeln einen letzten zweiten dichroitischen Spiegel, der vom ersten dichroitischen Spiegel (130, 230) entfernt ist, und mindestens einen vorhergehenden zweiten dichroitischen Spiegel umfasst, der sich zwischen dem letzten zweiten dichroitischen Spiegel und dem ersten dichroitischen Spiegel (130, 230) befindet, wobei die Vielzahl von Fluoreszenzen, die vom ersten dichroitischen Spiegel (130, 230) empfangen werden, sequentiell durch die Vielzahl von zweiten dichroitischen Spiegeln propagieren;
jeder vorhergehende zweite dichroitische Spiegel des/der vorhergehenden zweiten dichroitischen Spiegel(s) so positioniert ist, dass er eine von mindestens einer darauf einfallenden Fluoreszenz unter der Vielzahl von Fluoreszenzen zu der dem vorhergehenden zweiten dichroitischen Spiegel entsprechenden Abbildungsvorrichtung führt und die verbleibende Fluoreszenz der mindestens einen Fluoreszenz zu einem nächsten zweiten dichroitischen Spiegel führt, der an den vorhergehenden zweiten dichroitischen Spiegel angrenzt; und
der letzte zweite dichroitische Spiegel so positioniert ist, dass er darauf einfallende Fluoreszenz zu der dem letzten zweiten dichroitischen Spiegel entsprechenden Abbildungsvorrichtung führt.

12. Die Detektionsvorrichtung (10, 20, 30) nach Anspruch 11, wobei der zweite dichroitische Spiegel, der dem ersten dichroitischen Spiegel (130, 230) unter dem mindestens einen vorhergehenden zweiten dichroitischen Spiegel am nächsten liegt, so ausgewählt ist, dass er die Fluoreszenz, die dadurch zu der entsprechenden Abbildungsvorrichtung geführt wird, durchlässt und die verbleibende Fluoreszenz der Vielzahl von darauf einfallenden Fluoreszenzen zu einem nächsten, daran angrenzenden zweiten dichroitischen Spiegel reflektiert, der letzte zweite dichroitische Spiegel so ausgewählt ist, dass er die darauf einfallende Fluoreszenz durch Reflexion zu der dem letzten zweiten dichroitischen Spiegel entsprechenden Abbildungsvorrichtung führt; und für jeden der zweiten dichroitischen Spiegel in der Vielzahl von zweiten dichroitischen Spiegeln, ausser dem dem ersten dichroitischen Spiegel (130, 230) am nächsten liegenden zweiten dichroitischen Spiegel und dem letzten zweiten dichroitischen Spiegel, der zweite dichroitische Spiegel so ausgewählt ist, dass er die Fluoreszenz, die von ihm zu der ihm entsprechenden Abbildungsvorrichtung geführt wird, reflektiert und die verbleibende Fluoreszenz der mindestens einen darauf einfallenden Fluoreszenz zu einem nächsten, daran angrenzenden zweiten dichroitischen Spiegel durchlässt; oder
wobei der zweite dichroitische Spiegel, der dem ersten dichroitischen Spiegel (130, 230) unter dem mindestens einen vorhergehenden zweiten dichroitischen Spiegel am nächsten liegt, so ausgewählt ist, dass er die Fluoreszenz, die dadurch zu der entsprechenden Abbildungsvorrichtung geführt wird, reflektiert und die verbleibende Fluoreszenz der Vielzahl von darauf einfallenden Fluoreszenzen zu einem nächsten, daran angrenzenden zweiten dichroitischen Spiegel durchlässt, der letzte zweite dichroitische Spiegel so ausgewählt ist, dass er die darauf einfallende Fluoreszenz durch Reflexion zu der dem letzten zweiten dichroitischen Spiegel entsprechenden Abbildungsvorrichtung führt; und für jeden der zweiten dichroitischen Spiegel in der Vielzahl von zweiten dichroitischen Spiegeln, ausser dem dem ersten dichroitischen Spiegel (130, 230) am nächsten liegenden zweiten dichroitischen Spiegel und dem letzten zweiten dichroitischen Spiegel, der zweite dichroitische Spiegel so ausgewählt ist, dass er die Fluoreszenz, die von ihm zu der ihm entsprechenden Abbildungsvorrichtung geführt wird, reflektiert und die verbleibende Fluoreszenz der mindestens einen darauf einfallenden Fluoreszenz zu einem nächsten, daran angrenzenden zweiten dichroitischen Spiegel durchlässt.

13. Ein Gensequenzierungssystem, umfassend ein Abbildungssystem (160, 260, 360) zum Sammeln eines Fluoreszenzsignals auf einem Sequenzierungschip; und ein optisches System, das sich zwischen dem Abbildungssystem (160, 260, 360) und dem Sequenzierungschip befindet, **dadurch gekennzeichnet, dass** das optische System umfasst:
eine Lichtquelle, die dazu konfiguriert ist, einen einfallenden Lichtstrahl einer Vielzahl von unterschiedlichen Wellenlängen zu emittieren;
eine Strahlteilervorrichtung (120), die dazu konfiguriert ist, den einfallenden Lichtstrahl einer Vielzahl unterschiedlicher Wellenlängen von der Lichtquelle zu empfangen und zu trennen, um eine Vielzahl von Anregungslichtstrahlen zu bilden, die der Vielzahl von unterschiedlichen Wellenlängen des einfallenden Lichtstrahls entsprechen, die in unterschiedlichen Emissionsrichtungen von der Strahlteilervorrichtung (120) emittiert werden, wobei jeder der Anregungslichtstrahlen eine Wellenlänge in einer Eins-zu-Eins-Korrespondenz mit der Vielzahl von unterschiedlichen Wellenlängen des einfallenden Lichtstrahls aufweist;
einen ersten dichroitischen Spiegel (130, 230), der dazu konfiguriert ist, die Vielzahl von Anregungslichtstrahlen zu empfangen, die der Vielzahl von unterschiedlichen Wellenlängen des einfallenden Lichtstrahls in einer Eins-zu-Eins-Korrespondenz entsprechen und von der Strahlteilervorrichtung (120) emittiert werden;
ein Objektiv (140, 240, 340), das zwischen dem Sequenzierungschip und dem ersten dichroitischen Spiegel (130, 230) angeordnet und dazu konfiguriert ist, die Vielzahl von Anregungslichtstrahlen zu empfangen, die durch den ersten dichroitischen Spiegel (130, 230) übertragen werden, um die Vielzahl von Anregungslichtstrahlen auf eine Vielzahl von unterschiedlichen Bereichen auf dem Sequenzierungschip zu fokussieren, um eine Vielzahl von Fluoreszenzen in der Vielzahl von unterschiedlichen Bereichen in Eins-zu-Eins-Korrespondenz mit der Vielzahl von Anregungslichtstrahlen jeweils anzuregen, und um die Vielzahl von Fluoreszenzen an den ersten dichroitischen Spiegel (130) zu übertragen; und
eine Fluoreszenzführungsvorrichtung (150, 250), die dazu konfiguriert ist, die Vielzahl von Fluoreszenzen zu empfangen, die über den ersten dichroitischen Spiegel (130, 230) übertragen werden, und die Vielzahl von Fluoreszenzen jeweils zu dem Abbildungssystem (160, 260, 360) zu führen,
wobei das Abbildungssystem (160, 260, 360) eine Vielzahl von Abbildungsvorrichtungen umfasst, die der Vielzahl von Fluoreszenzen in einer Eins-zu-Eins-Korrespondenz entsprechen, wobei jede der Vielzahl von Abbildungsvorrichtungen dazu konfiguriert ist, eine Fluoreszenz, die der Abbildungsvorrichtung entspricht, von der Vielzahl von Fluoreszenzen zu empfangen, die durch die Fluoreszenzführungsvorrichtung (150, 250) geführt werden, und die Fluoreszenz abzubilden, um Fluoreszenzinformationen zu erhalten, die der Fluoreszenz entsprechen,
wobei die Strahlteilervorrichtung (120) so ausgewählt ist, dass ein eingeschlossener Winkel zwischen Emissionsrichtungen von Anregungslichtstrahlen benachbarter Wellenlängen unter der Vielzahl von Anregungslichtstrahlen, die von der Strahlteilervorrichtung (120) emittiert werden, nicht kleiner ist als ein Schwellenwinkel,
wobei der Schwellenwinkel auf einem Verhältnis eines vorbestimmten Mindestabstands zu einer Brennweite des Objektivs (140, 240, 340) basiert, wobei der vorbestimmte Mindestabstand einen Mindestabstand angibt, bei dem Fokusflecken, die auf dem Sequenzierungschip durch die Vielzahl von Anregungslichtstrahlen gebildet werden, voneinander beabstandet sein müssen,
das optische System ferner umfasst:
eine zweite Strahlformungsvorrichtung (280), die sich zwischen der Strahlteilervorrichtung (120) und dem ersten dichroitischen Spiegel (130, 230) befindet und so positioniert ist, dass sie die Vielzahl von Anregungslichtstrahlen formt, die dem einfallenden Licht einer Vielzahl von unterschiedlichen Wellenlängen in einer Eins-zu-Eins-Korrespondenz entsprechen und von der Strahlteilervorrichtung (120) emittiert werden, und die geformte Vielzahl von Anregungslichtstrahlen an den ersten dichroitischen Spiegel (130, 230) überträgt,
wobei die zweite Strahlformungsvorrichtung (280) eine Winkelvergrösserung aufweist und der Schwellenwinkel gleich einem Produkt des Verhältnisses des vorbestimmten Mindestabstands zur Brennweite des Objektivs (140, 240, 340) multipliziert mit einem Koeffizienten ist, wobei der Koeffizient basierend auf der Winkelvergrösserung der zweiten Strahlformungsvorrichtung (280) bestimmt wird.

14. Ein Detektionsverfahren, umfassend:
Emittieren, durch eine Lichtquelle oder eine Lichtleitungsvorrichtung, eines einfallenden Lichtstrahls einer Vielzahl von unterschiedlichen Wellenlängen;
Empfangen und Trennen, durch eine Strahlteilervorrichtung (120), des einfallenden Lichtstrahls einer Vielzahl von unterschiedlichen Wellenlängen von der Lichtquelle oder der Lichtleitungsvorrichtung, um eine Vielzahl von Anregungslichtstrahlen zu bilden, die der Vielzahl von unterschiedlichen Wellenlängen des einfallenden Lichtstrahls entsprechen, die in unterschiedlichen Emissionsrichtungen von der Strahlteilervorrichtung (120) emittiert werden, wobei jeder der Anregungslichtstrahlen eine Wellenlänge in einer Eins-zu-Eins-Korrespondenz mit der Vielzahl von unterschiedlichen Wellenlängen des einfallenden Lichtstrahls aufweist;
Empfangen, durch einen ersten dichroitischen Spiegel (130, 230), der Vielzahl von Anregungslichtstrahlen, die der Vielzahl von unterschiedlichen Wellenlängen des einfallenden Lichtstrahls in einer Eins-zu-Eins-Korrespondenz entsprechen und von der Strahlteilervorrichtung (120) emittiert werden;
Empfangen, durch ein Objektiv (140, 240, 340), das zwischen einer zu detektierenden Probe (100, 200, 300) und dem ersten dichroitischen Spiegel (130, 230) angeordnet ist, der Vielzahl von Anregungslichtstrahlen, die durch den ersten dichroitischen Spiegel (130, 230) übertragen werden, Fokussieren, durch das Objektiv (140, 240, 340), der Vielzahl von Anregungslichtstrahlen auf eine Vielzahl von unterschiedlichen Bereichen auf der Probe (100, 200, 300), um eine Vielzahl von Fluoreszenzen in der Vielzahl von unterschiedlichen Bereichen in Eins-zu-Eins-Korrespondenz mit der Vielzahl von Anregungslichtstrahlen jeweils anzuregen, und Übertragen, durch das Objektiv (140, 240, 340), der Vielzahl von Fluoreszenzen an den ersten dichroitischen Spiegel (130, 230); und
Empfangen, durch eine Fluoreszenzführungsvorrichtung (150, 250), der Vielzahl von Fluoreszenzen, die durch den ersten dichroitischen Spiegel (130, 230) übertragen werden, und Führen, durch die Fluoreszenzführungsvorrichtung (150, 250), der Vielzahl von Fluoreszenzen zu einer Vielzahl von Abbildungsvorrichtungen, die der Vielzahl von Fluoreszenzen in einer Eins-zu-Eins-Korrespondenz entsprechen, so dass jede Fluoreszenz der Vielzahl von Fluoreszenzen von einer der Vielzahl von Abbildungsvorrichtungen, die der Fluoreszenz entsprechen, empfangen und abgebildet wird, um Fluoreszenzinformationen zu erhalten, die der Fluoreszenz zur Detektion entsprechen,
wobei die Strahlteilervorrichtung (120) so ausgewählt ist, dass ein eingeschlossener Winkel zwischen Emissionsrichtungen von Anregungslichtstrahlen benachbarter Wellenlängen unter der Vielzahl von Anregungslichtstrahlen, die von der Strahlteilervorrichtung (120) emittiert werden, nicht kleiner ist als ein Schwellenwinkel,
wobei der Schwellenwinkel auf einem Verhältnis eines vorbestimmten Mindestabstands zu einer Brennweite des Objektivs (140, 240, 340) basiert, wobei der vorbestimmte Mindestabstand einen Mindestabstand angibt, bei dem Fokusflecken, die auf der Probe (100, 200, 300) durch die Vielzahl von Anregungslichtstrahlen gebildet werden, voneinander beabstandet sein müssen,
das Detektionsverfahren ferner umfasst:
Formen, durch eine zweite Strahlformungsvorrichtung (280), die sich zwischen der Strahlteilervorrichtung (120) und dem ersten dichroitischen Spiegel (130, 230) befindet, der Vielzahl von Anregungslichtstrahlen, die dem einfallenden Lichtstrahl einer Vielzahl von unterschiedlichen Wellenlängen in einer Eins-zu-Eins-Korrespondenz entsprechen und von der Strahlteilervorrichtung (120) emittiert werden, und Übertragen, durch die zweite Strahlformungsvorrichtung (280), der geformten Vielzahl von Anregungslichtstrahlen an den ersten dichroitischen Spiegel (130, 230),
wobei die zweite Strahlformungsvorrichtung (280) eine Winkelvergrösserung aufweist und der Schwellenwinkel gleich einem Produkt des Verhältnisses des vorbestimmten Mindestabstands zur Brennweite des Objektivs (140, 240, 340) multipliziert mit einem Koeffizienten ist, wobei der Koeffizient basierend auf der Winkelvergrösserung der zweiten Strahlformungsvorrichtung (280) bestimmt wird.

## Revendications

1. Un appareil de détection (10, 20, 30), comprenant un dispositif de séparation de faisceau (120), un premier miroir dichroïque (130, 230), une lentille d'objectif (140, 240, 340), un dispositif de guidage de fluorescence (150, 250), et un système d'imagerie (160, 260, 360) comprenant une pluralité de dispositifs d'imagerie, dans lequel,
le dispositif de séparation de faisceau (120) est configuré pour recevoir et séparer un faisceau lumineux incident d'une pluralité de longueurs d'onde différentes provenant d'une fibre optique (210), pour former une pluralité de faisceaux lumineux d'excitation correspondant à la pluralité de longueurs d'onde différentes du faisceau lumineux incident émis dans différentes directions d'émission depuis le dispositif de séparation de faisceau (120), dans lequel, chacun des faisceaux lumineux d'excitation a une longueur d'onde en correspondance univoque avec la pluralité de longueurs d'onde différentes du faisceau lumineux incident;
le premier miroir dichroïque (130, 230) est positionné pour recevoir la pluralité de faisceaux lumineux d'excitation correspondant à la pluralité de longueurs d'onde différentes du faisceau lumineux incident en correspondance univoque et émis par le dispositif de séparation de faisceau (120), pour transmettre la pluralité de faisceaux lumineux d'excitation à la lentille d'objectif (140, 240, 340), de sorte que la pluralité de faisceaux lumineux d'excitation soient focalisés respectivement sur une pluralité de zones différentes d'un échantillon (100, 200, 300) à détecter à travers la lentille d'objectif (140, 240, 340), pour exciter une pluralité de fluorescences dans la pluralité de zones différentes de l'échantillon (100, 200, 300) sur lesquelles la pluralité de faisceaux lumineux d'excitation sont focalisés respectivement, et pour recevoir la pluralité de fluorescences excitées par la pluralité de faisceaux lumineux d'excitation respectivement et transmettre la pluralité de fluorescences au dispositif de guidage de fluorescence (150, 250);
la lentille d'objectif (140, 240, 340) est positionnée pour recevoir la pluralité de faisceaux lumineux d'excitation transmis par le premier miroir dichroïque (130, 230), pour focaliser la pluralité de faisceaux lumineux d'excitation sur la pluralité de zones différentes de l'échantillon (100, 200, 300) respectivement, et pour transmettre la pluralité de fluorescences excitées par la pluralité de faisceaux lumineux d'excitation respectivement au premier miroir dichroïque (130, 230);
le dispositif de guidage de fluorescence (150, 250) est positionné pour recevoir la pluralité de fluorescences transmises par le premier miroir dichroïque (130, 230), et pour guider la pluralité de fluorescences vers la pluralité de dispositifs d'imagerie respectivement, de sorte que chaque fluorescence de la pluralité de fluorescences soit imagée par l'un de la pluralité de dispositifs d'imagerie correspondant à la fluorescence; et
chacun de la pluralité de dispositifs d'imagerie est positionné pour recevoir une fluorescence de la pluralité de fluorescences guidées par le dispositif de guidage de fluorescence (150, 250), et pour imager la fluorescence pour obtenir des informations de fluorescence correspondant à la fluorescence pour la détection,
dans lequel le dispositif de séparation de faisceau (120) est choisi de telle manière qu'un angle inclus entre les directions d'émission des faisceaux lumineux d'excitation de longueurs d'onde adjacentes parmi la pluralité de faisceaux lumineux d'excitation émis par le dispositif de séparation de faisceau (120) n'est pas inférieur à un angle seuil,
dans lequel l'angle seuil est basé sur un rapport d'un espacement minimum prédéterminé à une distance focale de la lentille d'objectif (140, 240, 340), l'espacement minimum prédéterminé indiquant une distance minimale à laquelle les points de focalisation formés sur l'échantillon (100, 200, 300) par la pluralité de faisceaux lumineux d'excitation doivent être espacés les uns des autres,
l'appareil de détection comprend en outre:
un deuxième dispositif de mise en forme de faisceau (280) situé entre le dispositif de séparation de faisceau (120) et le premier miroir dichroïque (130, 230), et étant positionné pour mettre en forme la pluralité de faisceaux lumineux d'excitation correspondant à la lumière incidente d'une pluralité de longueurs d'onde différentes en correspondance univoque et émis par le dispositif de séparation de faisceau (120), et pour transmettre la pluralité de faisceaux lumineux d'excitation mis en forme au premier miroir dichroïque (130, 230),
dans lequel le deuxième dispositif de mise en forme de faisceau (280) a un grossissement angulaire, et l'angle seuil est égal à un produit du rapport de l'espacement minimum prédéterminé à la distance focale de la lentille d'objectif (140, 240, 340) multiplié par un coefficient, le coefficient étant déterminé en fonction du grossissement angulaire du deuxième dispositif de mise en forme de faisceau (280).

2. L'appareil de détection (10, 20, 30) selon la revendication 1, comprenant en outre:
au moins une source lumineuse configurée pour générer une pluralité de lumières d'excitation de différentes longueurs d'onde,
dans lequel la fibre optique (210) est positionnée pour recevoir la pluralité de lumières d'excitation de différentes longueurs d'onde de l'au moins une source lumineuse, et pour former le faisceau lumineux incident d'une pluralité de longueurs d'onde différentes et transmettre le faisceau lumineux incident d'une pluralité de longueurs d'onde différentes au dispositif de séparation de faisceau (120).

3. L'appareil de détection (20) selon la revendication 1, comprenant en outre:
un premier dispositif de mise en forme de faisceau (270) situé entre la fibre optique (210) et le dispositif de séparation de faisceau (120), et étant positionné pour mettre en forme le faisceau lumineux incident d'une pluralité de longueurs d'onde différentes sortant de la fibre optique (210), et pour transmettre le faisceau lumineux incident mis en forme au dispositif de séparation de faisceau (120).

4. L'appareil de détection (10, 20, 30) selon la revendication 1, dans lequel le coefficient est égal à l'inverse d'une valeur absolue du grossissement angulaire.

5. L'appareil de détection (10, 20, 30) selon la revendication 1, dans lequel le dispositif de séparation de faisceau (120) comprend au moins un prisme de dispersion (220), ou dans lequel le dispositif de séparation de faisceau (120) comprend au moins un réseau.

6. L'appareil de détection (10, 20, 30) selon la revendication 5, dans lequel l'au moins un prisme de dispersion (320) comprend un seul prisme de dispersion (320) positionné de telle manière que le faisceau lumineux incident d'une pluralité de longueurs d'onde différentes provenant de la fibre optique (210) est incident sur une première surface réfractive (3201) du seul prisme de dispersion (320) et chacun de la pluralité de faisceaux lumineux d'excitation sort ensuite d'une deuxième surface réfractive (3202) qui est différente de la première surface réfractive (3201), du seul prisme de dispersion (320).

7. L'appareil de détection (10, 20, 30) selon la revendication 6, dans lequel le faisceau lumineux incident d'une pluralité de longueurs d'onde différentes est incident sur la première surface réfractive (3201) du seul prisme de dispersion (320) à un angle d'incidence prédéterminé, l'angle d'incidence prédéterminé étant choisi de telle manière qu'un angle de déviation entre une direction d'incidence du faisceau lumineux incident sur la première surface réfractive (3201) et une direction de sortie de la pluralité de faisceaux lumineux d'excitation correspondant au faisceau lumineux incident et sortant de la deuxième surface réfractive (3202) est minimisé.

8. L'appareil de détection (10, 20, 30) selon la revendication 6, dans lequel la première surface réfractive (3201) et la deuxième surface réfractive (3202) sont des surfaces adjacentes du seul prisme de dispersion (320), le seul prisme de dispersion (320) a un angle au sommet formé entre la première surface réfractive (3201) et la deuxième surface réfractive (3202), et le seul prisme de dispersion (320) est choisi de telle manière qu'une combinaison de l'angle au sommet du seul prisme de dispersion (320) et d'un matériau choisi pour former le seul prisme de dispersion (320) permet que l'angle inclus ne soit pas inférieur à l'angle seuil.

9. L'appareil de détection (10, 20, 30) selon la revendication 5, dans lequel l'au moins un prisme de dispersion (320) comprend une pluralité de prismes de dispersion (320) qui sont agencés séquentiellement, chacun de la pluralité de prismes de dispersion (320) ayant un angle au sommet formé entre une première surface réfractive (3201) et une deuxième surface réfractive (3202) de celui-ci qui sont adjacentes et étant positionné pour recevoir chaque lumière incidente sur celui-ci avec sa première surface réfractive (3201) et faire sortir la lumière de sa deuxième surface réfractive (3202), dans lequel une combinaison d'un nombre de la pluralité de prismes de dispersion (320) et d'un angle au sommet et d'un matériau de chacun de la pluralité de prismes de dispersion est choisie de telle manière que l'angle inclus ne soit pas inférieur à l'angle seuil.

10. L'appareil de détection (10, 20, 30) selon la revendication 9, dans lequel un premier prisme de dispersion (320) parmi la pluralité de prismes de dispersion (320) qui reçoit en premier le faisceau lumineux incident d'une pluralité de longueurs d'onde différentes provenant de la fibre optique (210) est positionné de telle manière que le faisceau lumineux incident d'une pluralité de longueurs d'onde différentes provenant de la fibre optique (210) est incident sur une première surface réfractive (3201) du premier prisme de dispersion (320) et sort ensuite d'une deuxième surface réfractive (3202) du premier prisme de dispersion (320), et chacun de la pluralité de prismes de dispersion (320) autre que le premier prisme de dispersion (320) est positionné de telle manière que chaque lumière sortant d'un prisme de dispersion précédent (320) adjacent à celui-ci est incidente sur une première surface réfractive (3201) de celui-ci et sort d'une deuxième surface réfractive (3202) de celui-ci.

11. L'appareil de détection (10, 20, 30) selon la revendication 1, dans lequel le dispositif de guidage de fluorescence (150, 250) comprend une pluralité de deuxièmes miroirs dichroïques qui sont agencés séquentiellement, la pluralité de deuxièmes miroirs dichroïques comprenant un dernier deuxième miroir dichroïque qui est éloigné du premier miroir dichroïque (130, 230) et au moins un deuxième miroir dichroïque précédent situé entre le dernier deuxième miroir dichroïque et le premier miroir dichroïque (130, 230), la pluralité de fluorescences reçues par le premier miroir dichroïque (130, 230) se propageant séquentiellement à travers la pluralité de deuxièmes miroirs dichroïques;
chaque deuxième miroir dichroïque précédent du ou des deuxièmes miroirs dichroïques précédents est positionné pour guider l'une d'au moins une fluorescence incidente sur celui-ci parmi la pluralité de fluorescences vers le dispositif d'imagerie correspondant au deuxième miroir dichroïque précédent, et pour guider les fluorescences restantes de l'au moins une fluorescence vers un deuxième miroir dichroïque suivant adjacent au deuxième miroir dichroïque précédent; et
le dernier deuxième miroir dichroïque est positionné pour guider la fluorescence incidente sur celui-ci vers le dispositif d'imagerie correspondant au dernier deuxième miroir dichroïque.

12. L'appareil de détection (10, 20, 30) selon la revendication 11, dans lequel le deuxième miroir dichroïque situé le plus près du premier miroir dichroïque (130, 230) parmi l'au moins un deuxième miroir dichroïque précédent est choisi pour transmettre la fluorescence qui est guidée par celui-ci vers le dispositif d'imagerie correspondant à celui-ci et réfléchir les fluorescences restantes de la pluralité de fluorescences incidentes sur celui-ci vers un deuxième miroir dichroïque suivant qui lui est adjacent, le dernier deuxième miroir dichroïque est choisi pour guider la fluorescence incidente sur celui-ci vers le dispositif d'imagerie correspondant au dernier deuxième miroir dichroïque par réflexion; et pour chacun du ou des deuxièmes miroirs dichroïques dans la pluralité de deuxièmes miroirs dichroïques autres que le deuxième miroir dichroïque situé le plus près du premier miroir dichroïque (130, 230) et le dernier deuxième miroir dichroïque, le deuxième miroir dichroïque est choisi pour réfléchir la fluorescence qui est guidée par celui-ci vers le dispositif d'imagerie correspondant à celui-ci et transmettre les fluorescences restantes de l'au moins une fluorescence incidente sur celui-ci vers un deuxième miroir dichroïque suivant qui lui est adjacent; ou
dans lequel le deuxième miroir dichroïque situé le plus près du premier miroir dichroïque (130, 230) parmi l'au moins un deuxième miroir dichroïque précédent est choisi pour réfléchir la fluorescence qui est guidée par celui-ci vers le dispositif d'imagerie correspondant à celui-ci et transmettre les fluorescences restantes de la pluralité de fluorescences incidentes sur celui-ci vers un deuxième miroir dichroïque suivant qui lui est adjacent, le dernier deuxième miroir dichroïque est choisi pour guider la fluorescence incidente sur celui-ci vers le dispositif d'imagerie correspondant au dernier deuxième miroir dichroïque par réflexion; et pour chacun du ou des deuxièmes miroirs dichroïques dans la pluralité de deuxièmes miroirs dichroïques autres que le deuxième miroir dichroïque situé le plus près du premier miroir dichroïque (130, 230) et le dernier deuxième miroir dichroïque, le deuxième miroir dichroïque est choisi pour réfléchir la fluorescence qui est guidée par celui-ci vers le dispositif d'imagerie correspondant à celui-ci et transmettre les fluorescences restantes de l'au moins une fluorescence incidente sur celui-ci vers un deuxième miroir dichroïque suivant qui lui est adjacent.

13. Un système de séquençage génétique, comprenant un système d'imagerie (160, 260, 360) pour collecter un signal de fluorescence sur une puce de séquençage; et un système optique situé entre le système d'imagerie (160, 260, 360) et la puce de séquençage, **caractérisé en ce que** le système optique comprend:
une source lumineuse configurée pour émettre un faisceau lumineux incident d'une pluralité de longueurs d'onde différentes;
un dispositif de séparation de faisceau (120) configuré pour recevoir et séparer le faisceau lumineux incident d'une pluralité de longueurs d'onde différentes de la source lumineuse, pour former une pluralité de faisceaux lumineux d'excitation correspondant à la pluralité de longueurs d'onde différentes du faisceau lumineux incident émis dans différentes directions d'émission depuis le dispositif de séparation de faisceau (120), dans lequel, chacun des faisceaux lumineux d'excitation a une longueur d'onde en correspondance univoque avec la pluralité de longueurs d'onde différentes du faisceau lumineux incident;
un premier miroir dichroïque (130, 230) configuré pour recevoir la pluralité de faisceaux lumineux d'excitation correspondant à la pluralité de longueurs d'onde différentes du faisceau lumineux incident en correspondance univoque et émis par le dispositif de séparation de faisceau (120);
une lentille d'objectif (140, 240, 340) agencée entre la puce de séquençage et le premier miroir dichroïque (130, 230) et configurée pour recevoir la pluralité de faisceaux lumineux d'excitation transmis par le premier miroir dichroïque (130, 230), pour focaliser la pluralité de faisceaux lumineux d'excitation sur une pluralité de zones différentes sur la puce de séquençage pour exciter une pluralité de fluorescences dans la pluralité de zones différentes en correspondance univoque avec la pluralité de faisceaux lumineux d'excitation respectivement, et pour transmettre la pluralité de fluorescences au premier miroir dichroïque (130); et
un dispositif de guidage de fluorescence (150, 250) configuré pour recevoir la pluralité de fluorescences transmises via le premier miroir dichroïque (130, 230) et pour guider la pluralité de fluorescences vers le système d'imagerie (160, 260, 360) respectivement,
dans lequel le système d'imagerie (160, 260, 360) comprend une pluralité de dispositifs d'imagerie correspondant à la pluralité de fluorescences en correspondance univoque, chacun de la pluralité de dispositifs d'imagerie étant configuré pour recevoir une fluorescence, qui correspond au dispositif d'imagerie, de la pluralité de fluorescences guidées par le dispositif de guidage de fluorescence (150, 250), et pour imager la fluorescence afin d'obtenir des informations de fluorescence correspondant à la fluorescence,
dans lequel le dispositif de séparation de faisceau (120) est choisi de telle manière qu'un angle inclus entre les directions d'émission des faisceaux lumineux d'excitation de longueurs d'onde adjacentes parmi la pluralité de faisceaux lumineux d'excitation émis par le dispositif de séparation de faisceau (120) n'est pas inférieur à un angle seuil,
dans lequel l'angle seuil est basé sur un rapport d'un espacement minimum prédéterminé à une distance focale de la lentille d'objectif (140, 240, 340), l'espacement minimum prédéterminé indiquant une distance minimale à laquelle les points de focalisation formés sur la puce de séquençage par la pluralité de faisceaux lumineux d'excitation doivent être espacés les uns des autres,
le système optique comprend en outre:
un deuxième dispositif de mise en forme de faisceau (280) situé entre le dispositif de séparation de faisceau (120) et le premier miroir dichroïque (130, 230), et étant positionné pour mettre en forme la pluralité de faisceaux lumineux d'excitation correspondant à la lumière incidente d'une pluralité de longueurs d'onde différentes en correspondance univoque et émis par le dispositif de séparation de faisceau (120), et pour transmettre la pluralité de faisceaux lumineux d'excitation mis en forme au premier miroir dichroïque (130, 230),
dans lequel le deuxième dispositif de mise en forme de faisceau (280) a un grossissement angulaire, et l'angle seuil est égal à un produit du rapport de l'espacement minimum prédéterminé à la distance focale de la lentille d'objectif (140, 240, 340) multiplié par un coefficient, le coefficient étant déterminé en fonction du grossissement angulaire du deuxième dispositif de mise en forme de faisceau (280).

14. Une méthode de détection, comprenant:
l'émission, par une source lumineuse ou un dispositif de conduction de lumière, d'un faisceau lumineux incident d'une pluralité de longueurs d'onde différentes;
la réception et la séparation, par un dispositif de séparation de faisceau (120), du faisceau lumineux incident d'une pluralité de longueurs d'onde différentes de la source lumineuse ou du dispositif de conduction de lumière, pour former une pluralité de faisceaux lumineux d'excitation correspondant à la pluralité de longueurs d'onde différentes du faisceau lumineux incident émis dans différentes directions d'émission depuis le dispositif de séparation de faisceau (120), dans lequel, chacun des faisceaux lumineux d'excitation a une longueur d'onde en correspondance univoque avec la pluralité de longueurs d'onde différentes du faisceau lumineux incident;
la réception, par un premier miroir dichroïque (130, 230), de la pluralité de faisceaux lumineux d'excitation correspondant à la pluralité de longueurs d'onde différentes du faisceau lumineux incident en correspondance univoque et émis par le dispositif de séparation de faisceau (120);
la réception, par une lentille d'objectif (140, 240, 340) agencée entre un échantillon (100, 200, 300) à détecter et le premier miroir dichroïque (130, 230), de la pluralité de faisceaux lumineux d'excitation transmis par le premier miroir dichroïque (130, 230), la focalisation, par la lentille d'objectif (140, 240, 340), de la pluralité de faisceaux lumineux d'excitation sur une pluralité de zones différentes sur l'échantillon (100, 200, 300) pour exciter une pluralité de fluorescences dans la pluralité de zones différentes en correspondance univoque avec la pluralité de faisceaux lumineux d'excitation respectivement, et la transmission, par la lentille d'objectif (140, 240, 340), de la pluralité de fluorescences au premier miroir dichroïque (130, 230); et
la réception, par un dispositif de guidage de fluorescence (150, 250), de la pluralité de fluorescences transmises par le premier miroir dichroïque (130, 230), et le guidage, par le dispositif de guidage de fluorescence (150, 250), de la pluralité de fluorescences vers une pluralité de dispositifs d'imagerie correspondant à la pluralité de fluorescences en correspondance univoque, de sorte que chaque fluorescence de la pluralité de fluorescences soit reçue et imagée par l'un de la pluralité de dispositifs d'imagerie correspondant à la fluorescence pour obtenir des informations de fluorescence correspondant à la fluorescence pour la détection,
dans lequel le dispositif de séparation de faisceau (120) est choisi de telle manière qu'un angle inclus entre les directions d'émission des faisceaux lumineux d'excitation de longueurs d'onde adjacentes parmi la pluralité de faisceaux lumineux d'excitation émis par le dispositif de séparation de faisceau (120) n'est pas inférieur à un angle seuil,
dans lequel l'angle seuil est basé sur un rapport d'un espacement minimum prédéterminé à une distance focale de la lentille d'objectif (140, 240, 340), l'espacement minimum prédéterminé indiquant une distance minimale à laquelle les points de focalisation formés sur l'échantillon (100, 200, 300) par la pluralité de faisceaux lumineux d'excitation doivent être espacés les uns des autres,
la méthode de détection comprend en outre:
la mise en forme, par un deuxième dispositif de mise en forme de faisceau (280) situé entre le dispositif de séparation de faisceau (120) et le premier miroir dichroïque (130, 230), de la pluralité de faisceaux lumineux d'excitation correspondant au faisceau lumineux incident d'une pluralité de longueurs d'onde différentes en correspondance univoque et émis par le dispositif de séparation de faisceau (120), et la transmission, par le deuxième dispositif de mise en forme de faisceau (280), de la pluralité de faisceaux lumineux d'excitation mis en forme au premier miroir dichroïque (130, 230),
dans lequel le deuxième dispositif de mise en forme de faisceau (280) a un grossissement angulaire, et l'angle seuil est égal à un produit du rapport de l'espacement minimum prédéterminé à la distance focale de la lentille d'objectif (140, 240, 340) multiplié par un coefficient, le coefficient étant déterminé en fonction du grossissement angulaire du deuxième dispositif de mise en forme de faisceau (280).
